(19) Europäisches Patentamt
European Patent Office
Office européen des brevets

(11) EP 4 389 263 A1

(12) EUROPEAN PATENT APPLICATION

(43) Date of publication:
**26.06.2024 Bulletin 2024/26**

(21) Application number: **22307013.7**

(22) Date of filing: **22.12.2022**

(51) International Patent Classification (IPC):
***B01D 61/00*** $^{(2006.01)}$ ***C07D 259/00*** $^{(2006.01)}$

(52) Cooperative Patent Classification (CPC):
**C07D 259/00;** B01D 67/0006; B01D 71/60; B01D 2323/30

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
- **Centre national de la recherche scientifique 75016 Paris (FR)**
- **UNIVERSITE DE MONTPELLIER 34090 Montpellier (FR)**
- **Ecole Nationale Supérieure de Chimie de Montpellier 34090 Montpellier (FR)**

(72) Inventors:
- **BENKHALED, Belkacem Tarek 34090 MONTPELLIER (FR)**
- **CHAIX, Arnaud 34430 SAINT JEAN DE VEDAS (FR)**
- **GOMRI, Chaimaa 34000 MONTPELLIER (FR)**
- **SEMSARILAR, Mona 34080 MONTPELLIER (FR)**
- **BLANQUER, Sébastien 34070 MONTPELLIER (FR)**

(74) Representative: **Novagraaf Technologies**
**Bâtiment O2**
**2, rue Sarah Bernhardt**
**CS90017**
**92665 Asnières-sur-Seine Cedex (FR)**

(54) **SUBSTITUTED TRIANGLAMINE AND USES THEREOF**

(57) The present invention belongs to the field of water purification and decontamination.

The present invention relates to new versatile family of trianglamine based compound and their uses thereof as is or in the manufacture of membranes and gels useful to purify and decontaminate water.

EP 4 389 263 A1

## Description

### Technical field

The present invention belongs to the field of water purification and decontamination.
The present invention relates to new versatile family of trianglamine based compound and their uses thereof as is or in the manufacture of membranes and gels useful to purify and decontaminate water.
In the description below, references between **[]** refer to the list of references at the end of the examples.

### Technical background

Nuclear energy contributes about 10% of the world's electricity production (~ 413 GW nowadays) and electricity production is in constant growth. The International Energy Agency (IEA) predicts about $1.1 trillion in investment in nuclear plants by 2040, representing 46% increase in nuclear power generation worldwide. In addition, to provide an effective response to major challenges facing the world such as greenhouse gas emissions, growing demand for electricity and the need for nations to have self-sufficient energy, nuclear energy is needed to play a crucial role to faces these challenges. Indeed, the growing need of nuclear energy led to a fast expand of nuclear plants, 56 nuclear plants under construction, 147 planned for construction, and with 436 actual nuclear plants in operation worldwide. For this purpose, it is urgent to propose innovative, effective, and low-cost solutions to prevent any major risks of pollution from nuclear waste.
Past nuclear power plant disasters, for example, Chernobyl nuclear accident on April 26, 1986, caused by a sudden and uncontrolled increase of the nuclear reaction led to the explosion of the reactor core and the destruction of the building, releasing into the atmosphere a pulse of a large number of radionuclides. The Fukushima Daiichi nuclear accident triggered by an Earthquake and tsunami struck on March 11, 2011, caused widespread contamination of areas with radionuclides such as $^{137}Cs$, $^{134}Cs$ $^{90}Sr$ and $^{131}I$. Nishikawa and co-workers reported in their study that the amount of radioactive iodine release after Fukushima disaster (~520 PBq) had been estimated to be about 10% compared to Chernobyl accident (~5300 PBq). Furthermore, there have been other major nuclear accidents in the last fifty years (the K-19 in the North Atlantic in 1961, the explosion of the Kyshtym nuclear power plant in 1975, Three Mile Island in the United States in 1979, e.g.) and several other accidents related to the release of radionuclides into the environment.
Nuclear waste substances, such as radioactive iodine produced from uranium fission, releases volatile solid gases and can easily dissolve in water, posing serious threat to the public and the ecosystem. Radioiodine such as $^{131}I$ and $^{129}I$ are the two main components of nuclear waste, with half-lives of 8.02 days and 15.7 million years, respectively. Moreover, in the waste stream of nuclear plant $^{131}I$ exist in various forms, including molecular iodine ($I_2$), iodide ($I^-$), hypoiodite ion ($IO^-$), and iodate ($IO_3^-$).
For this purpose, radioiodine environmental contamination attracts strong public concern due to the highly volatile nature of radioactive iodine with fast diffusion into air and water, leading to radiological contamination of the environment and affecting the population's health. The long-term exposition (inhaled or swallowed) of radioiodine and even at a low dose, binds to the thyroid gland and can cause severe tissue damage and thyroid cancer. It is therefore imperative, that once a nuclear accident or leak occurs to urgently resolve and intervene to capture the aqueous and volatile radioactive iodine to prevent contaminations of the ecosystem. In order to contain such accidents or leakage from nuclear plant, researchers have increasingly focused on developing materials for the capture and storage of radioactive iodine from aqueous and vapors.
To date, the industrial technology to remove radioiodine is based on the chemical transformation of iodine into AgI using silver-doped adsorbents such as natural or synthetic zeolites, $Ag^0$ faujasite or modernite and used as benchmark materials. Since decades, a large number of potential iodine adsorbents have been explored, including silica-coated magnetite nanoparticles, zérovalent iron nanoparticles, Mg-Al($NO_3$) layered double hydroxide (LDH), activated carbon, graphene-based sorbents, $Ag_2O$ grafted titanate nanolamina, and layered bismuth-iodine-oxide.
However, most of these sorbents suffered from low and slow adsorption capacity and inefficient recovery, prompting the development of alternative materials. Over the past few decades, progress in chemistry by researchers has led to the discovery of promising new porous materials as alternatives to the traditional materials mentioned above. These porous materials such as metal organic frameworks (MOFs), covalent organic frameworks (COFs) and porous organic polymers (POPs) with high and fast adsorption capacity, high specific surface area, high porosity, and good recycling capacity for addressing effective iodine capture from solutions and vapor phase. However, Valizadeh and co-workers briefly remind the major issues of these new porous materials for industrial applications, such as pressure drop, dustiness, clogging, mass loss, and challenges in handling and transportation **[1].**
Additionally, MOFs and COFs are still new laboratory materials and are prepared with very low amount and at high prices. In order to circumvent the drawback of powder of these adsorbents, cross-linked gels based on the combination of macrocycles and polymers represent an interesting and promising alternative. Macrocycles are crucial in many

applications such as molecular separation, adsorption, catalysis, sensors, and drug delivery. In the scientific literature, a catalog of several types of synthetic macrocycles has been designed, such as crown ethers, calixarene, cucurbituril, pillararenes, and many others. As an alternative to the inherent issues of certain macrocycles, trianglamines as definite and incomparable advantages due to quick preparation, easy-to-implement purification, and scalable at relatively low cost, making them an exceptional candidate so far for iodine capture. Furthermore, trianglamine is a macrocycle with guest recognition properties and have been used for several applications such as gas capture/separation, sensors, membranes, and host-guest selectivity. However, the handling of trianglamine powder for radionuclides capture is impractical due to its hydrophobicity and the difficulties of using a powder form in case of nuclear waste.

More recently, research work by many groups has led to the preparation of promising new materials as non-porous adaptive crystal, intrinsically porous materials (IPMs), organic cages and organogel, all of which based on macrocycles and have emerged as versatile platform for iodine capture.

Molecularly porous cross-linked membranes made from crosslinked trianglamines are known from WO2022/58961 **[2].** They are prepared by interfacial polymerization of a reactive macrocycle monomer with intrinsic microporous structure are provided. Macrocycles with multiple reacting sites for cross-linking provide a hyper-cross-linked network suitable for molecular separations employing polar or apolar solvents including organic solvent nanofiltration (OSN).

There is thus a need for membranes, that are inexpensive, easy to synthesize and produce, via a quick process.

There is a need for membranes whose size and thickness can be easily controlled.

The compound according to the invention can be fabricated by bulk polymerization of a mixture of a functionalized trianglamine with various monomers or multifunctional cross-links, bearing polymerizable functions such as acrylate, methacrylate or vinyl groups.

There is also a need for membranes having high mechanical properties, high thermal and chemical stability.

3D printing manifests divergent development in different industries and scholastic research. Professionals from various sectors are utilizing the 3D Printing technique over conventional ones because it is more affordable, user-friendly, and capable to manufacture complex structures rapidly with minimum wastage. 3D printed spacer with modified geometry shows better antifouling ability than conventional ones. Furthermore, 3D printed materials with tunable properties such as surface area, thickness, and roughness enhance the removal efficiency for heavy metals, oil, and organic matter from water compared to standard treatment techniques.

There is thus also a need for a membrane that can be easily 3D printed.

## Definitions

As used herein other than the claims, the terms "a," "an," "the," and/or "said" means one or more. As used herein in the claim(s), when used in conjunction with the words "comprise," "comprises" and/or "comprising," the words "a," "an," "the," and/or "said" may mean one or more than one. As used herein and in the claims, the terms "having," "has," "is," "have," "including," "includes," and/or "include" has the same meaning as "comprising," "comprises," and "comprise." As used herein and in the claims "another" may mean at least a second or more. As used herein and in the claims, "about" refers to any inherent measurement error or a rounding of digits for a value (e.g., a measured value, calculated value such as a ratio), and thus the term "about" may be used with any value and/or range.

The phrase "a combination thereof" "a mixture thereof" and such like following a listing, the use of "and/or" as part of a listing, a listing in a table, the use of "etc" as part of a listing, the phrase "such as," and/or a listing within brackets with "e.g.," or i.e., refers to any combination (e.g., any subset) of a set of listed components, and combinations and/or mixtures of related species and/or embodiments described herein though not directly placed in such a listing are also contemplated. Such related and/or like genera(s), sub-genera(s), specie(s), and/or embodiment(s) described herein are contemplated both in the form of an individual component that may be claimed, as well as a mixture and/or a combination that may be described in the claims as "at least one selected from," "a mixture thereof" and/or "a combination thereof."

In general, the term "substituted" whether preceded by the term "optionally" or not, and substituents contained in formulae of this invention, refer to the replacement of hydrogen radicals in a given structure with the radical of a specified substituent. When more than one position in any given structure may be substituted with more than one substituent selected from a specified group, the substituent may be either the same or different at every position. As used herein, the term "substituted" is contemplated to include all permissible substituents of organic compounds.

As used herein, the "heteroatom" means a different from carbon or hydrogen. Heteroatoms are preferably chosen from nitrogen, oxygen, and silicon.

As used herein, the term "aliphatic" refers to acyclic or cyclic, but non-aromatic hydrocarbon compound or group.

As used herein, the term "alkyl", refers to straight and branched alkyl groups. An analogous convention applies to other generic terms such as "alkenyl", "alkynyl" and the like. In certain embodiments, as used herein, "lower alkyl" is used to indicate those alkyl groups (substituted, unsubstituted, branched or unbranched) having about 1-6 carbon atoms. Illustrative alkyl groups include, but are not limited to, for example, methyl, ethyl, n-propyl, isopropyl, allyl, n-butyl, sec-butyl, isobutyl, tert-butyl, n-pentyl, sec-pentyl, isopentyl, tert-pentyl, n-hexyl, sec-hexyl, moieties and the like, which again, may bear one or more substituents. Alkenyl groups include, but are not limited to, for example, ethenyl, propenyl, butenyl, 1-methyl-2-buten-l-yl, and the like. Representative alkynyl groups include, but are not limited to, ethynyl, 2-propynyl (propargy1), 1-propynyl and the like.

The term "cycloalkyl" refers to cyclic alkyls having 3 to 10 carbon atoms in single or multiple cyclic rings, preferably 5 to 6 carbon atoms in a single cyclic ring (e.g. cyclopropyl, cyclobutyl, cyclopentyl, cyclohexyl, cycloheptyl).

As used herein, the term "heteroalkyl" refers to an alkyl as defined above having at least one carbon atom replaced by a heteroatom. Nonlimiting examples of suitable heteroatoms include nitrogen, oxygen, and sulfur.

In general, the term "aromatic moiety" or "aryl", as used herein, refers to stable substituted or unsubstituted unsaturated mono- or polycyclic hydrocarbon moieties having preferably 3-14 carbon atoms, comprising at least one ring satisfying the Hackle rule for aromaticity. Examples of aromatic moieties include, but are not limited to, phenyl, indanyl, indenyl, naphthyl, phenanthryl and anthracyl. As used herein, the term "heteroaryl" refers to an aryl having at least one aromatic carbon atom in the ring structure replaced by a heteroatom.

As used herein, the term "aralkyl" or "alkylaryl or alkylheteroaryl" refer to an alkyl having at least one hydrogen atom replaced by an aryl or heteroaryl group.

The term "halogen" as used herein refers to an atom selected from fluorine, chlorine, bromine and iodine. "halo group" refers to a halogen substituent such as -F, -Br, -Cl or -I.

As used herein, the term "haloaryl" refers to an aryl or heteroaryl having at least one hydrogen atom replaced by a halogen.

As used herein, the term "alkoxy" refers to the group - OR, wherein R is an alkyl or heteroalkyl group. Preferably, alkoxy are -$OR^4$ groups in which $R^4$ is a $C_1$ to $C_4$ alkyl chain (preferably -OMe).

As used herein, "amine" and "amino" (and its protonated form) refer to both unsubstituted and substituted amines.

As used herein, the term "independently" refers to the fact that the substituents, atoms or moieties to which these terms refer, are selected from the list of variables independently from each other (i.e., they may be identical or the same).

As used herein, the term "and/or" means any one of the items, any combination of the items, or all of the items with which this term is associated.

As used herein, the term "gel" encompasses "organogel", a gel composed of a liquid organic phase within a three-dimensional, cross-linked network and "hydrogel", a gel which is a network of polymer chains that are hydrophilic, sometimes found as a colloidal gel in which water is the dispersion medium,

**Detailed description of the invention**

The Applicant has developed a new functionalized trianglamine able to achieve high capture of iodine in water, seawater, and air. These new functionalized trianglamine are versatile compound that present many advantages such as uptake of iodine in both aqueous and vapor phase. They are easy to synthetize, with high yields. The new functionalized trianglamine can be elaborated of various materials due to the ease of the functionalization, and be made as materials such as films, membranes, gels, organogels, hydrogels, 3D printed materials, nanoparticules, water soluble co-polymers. These properties can be adapted or modulated by playing on nature or the length of the cross-linker, to obtain more or less stiff membrane, hydrophilic or hydrophobic membranes, depending on the desired application (gas separation, removal of dyes and ions from water, desalination).

These materials have strong potential in water decontamination, for instance, the removal of dyes, metal ions, iodine, radionuclides, polyfluoroalkyl substances (PFASs), and desalination. Also, potential in gas separation such as the separation of $CO_2$, $N_2$, $NH_3$, $H_2$, $O_2$, $CH_4$.

A first object of the invention is a trianglamine based compound of formula I:

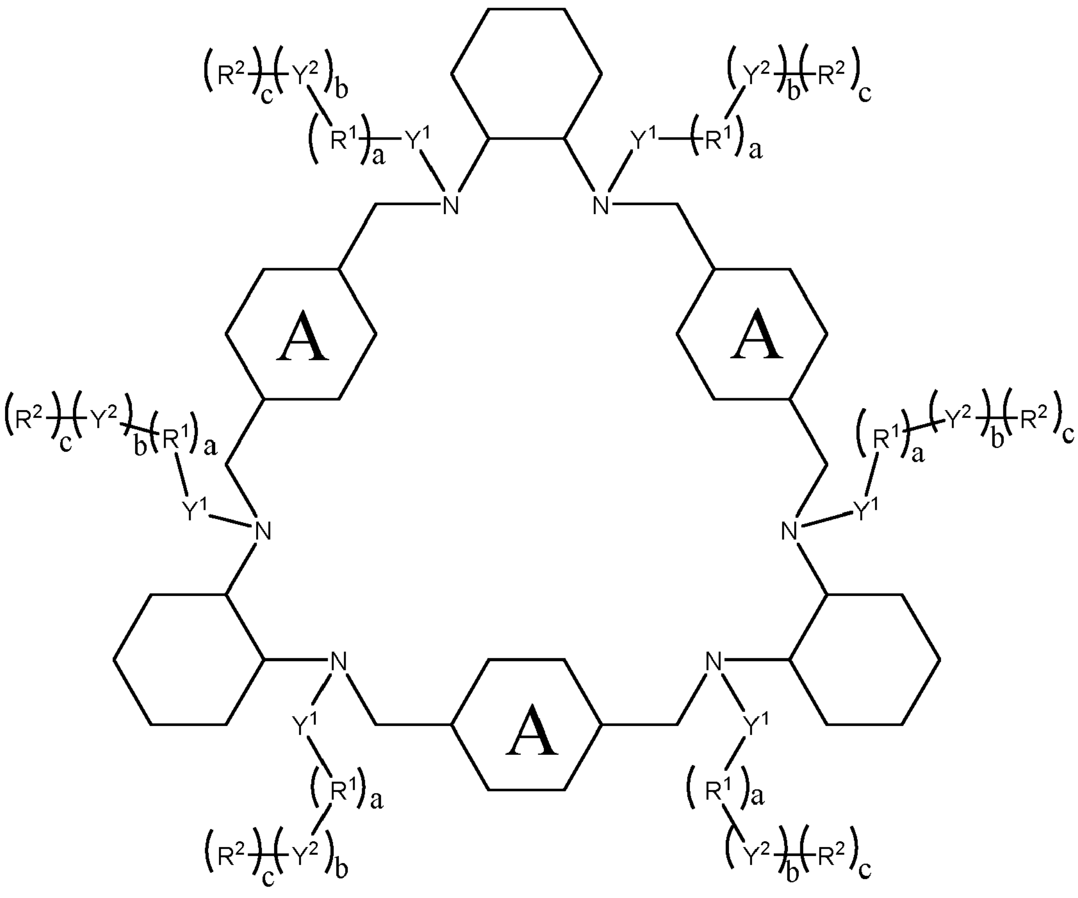

Formula I

wherein,

- each A independently represents a substituted or unsubstituted 4 to 18-membered aryl or heteroaryl linker,
- each $Y^1$ independently represents a bond or forms, together with the nitrogen of the trianglamine, a functional group chosen from urea (-N-C(O)-NH-), carbamate (-N-C(O)-O-) and amide (-N-C(O)-),
- each $R^1$ independently represents a mono or bifunctional radical chosen from H, a linear or cyclic, optionally branched, saturated or unsaturated, optionally substituted $C_1$ to $C_{30}$ hydrocarbon chain optionally comprising a heteroatom such as O or N, an optionally substituted $C_1$ to $C_{20}$ alkylaryl, an optionally substituted $C_1$ to $C_{20}$ alkylheteroaryl group, a substituted or unsubstituted $C_4$ to $C_6$-membered aryl or heteroaryl group or a polyether comprising from 2 to 100 monomers,
- at least one $-Y^1-(R^1)_a-(Y^2)_b-(R^2)_c$ radical is different from H,
- each a = 0 or 1,
- each b = 0 or 1,
- each c = 0 or 1,
- in a substituent $-Y^1-(R^1)_a-(Y^2)_b-(R^2)_c$ at least one from a, b and c is different from 0,
- each $Y^2$ independently represents a functional group chosen from hydroxyl (-OH), ester (-C(O)O- or -O-C(O)-), diester (-O-C(O)-$R^5$-C(O)-O-, in which $R^5$ is a $C_1$ to $C_8$ alkyl chain), carbamate (-NH-C(O)-O- or -O-C(O)-NH-), ether (-O-), amine (-NH- or -NR- in which R is a protecting group such as Boc), acrylate ($CH_2$=CHC(O)O-), methacrylate ($CH_2$=C($CH_3$)C(O)O-), -$SiOR^4_3$ in which $R^4$ is a $C_1$ to $C_4$ alkyl chain, halo group, a maleinimido group, a silicagel and a resin (such as polystyrene resin),
- each $R^2$ independently represents a linker of formula II:

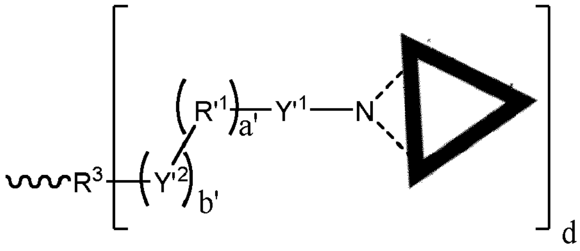

Formula II

wherein,

- $R^3$ represents a mono-, bi-, tri- or tetrafuntional radical comprising from 1 to 500 carbon atoms, optionally comprising hetero atoms such as O, N, Si or halo (e.g F, Br or I),
- the 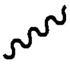 represents the point of attachment to $Y^2$ and

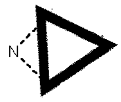

represents the point of attachment to another trianglamine based compound of formula I,
- $Y'^2$ is defined as $Y^2$, $R'^1$ is defined as $R^1$, $Y'^1$ is defined as $Y^1$, a' = 0 or 1, b' = 0 or 1 and d is an integer from 0 to 3.

Advantageously, $R^3$ is different from a cyclic alkyl or different from an aryl, when c = 1, d $\neq$ 0 and a = a' = b = b' = 0. Preferably, $R^3$ is different from di-, tri- or tetravalent phenyl, bi-phenyl, naphtyl, cyclopropyl, cyclobutyl, cyclopentyl, cyclohexyl or tetrahydrofuranyl group, when c = 1, d $\neq$ 0 and a = a' = b = b' = 0.

Advantageously, in a substituent $-Y^1-(R^1)_a-(Y^2)_b-(R^2)_c$, each a = 0 or 1, each b = 0 or 1 and each c = 0 or 1. At least one from a, b and c is different from 0. $Y'^2$ is defined as $Y^2$, $R'^1$ is defined as $R^1$, $Y'^1$ is defined as $Y^1$, a' = 0 or 1, b' = 0 or 1 and d is an integer from 0 to 3.

In a first variant of the invention, the compound of formula I is a crosslinked compound, when c = 1 and d = 1, 2 or 3. Preferably, when the compound of formula I is a crosslinked compound, $Y^1 = Y'^1$, $R^1 = R'^1$, $Y^2 = Y'^2$, a = a' and b = b'. A crosslinking rate is defined based on the number of N of the trianglamine bearing a substituent $-Y^1-(R^1)_a-(Y^2)_b-(R^2)_c$ different from H and connecting to another trianglamine (as represented in formula II) forming a network. Each trianglamine can be connected to up to 6 other trianglaine via the substituent $-Y^1-(R^1)_a-(Y^2)_b-(R^2)_c$ forming a network. The crosslinking rate can therefore be from 1/6 (about 16%) to 6/6 (100%). Depending on the application, the skilled person can choose the crosslinking rate.

In a second variant of the invention, the compound of formula I is a non-crosslinked compound, when c = 0 or when c = 1 and d = 0. A substitution rate is defined based on the number of N of the trianglamine bearing a substituent $-Y^1-(R^1)_a-(Y^2)_b-(R^2)_c$ different from H. The trianglamine can bear up to 6 substituents $-Y^1-(R^1)_a-(Y^2)_b-(R^2)_c$. The crosslinking rate can therefore be from 1/6 (about 16%) to 6/6 (100%). Depending on the application, the skilled person can choose the substitution rate.

In a third variant of the invention, the compound of formula I is a crosslinked compound, also bearing substituent not involved in forming the crosslinked network. Each trianglamine of the network may be substitute by a substituent in which c = 0, or c = 1 and d = 0 and by crosslinkers in which c = 1 and d = 1, 2 or 3. The crosslinking rate and substitution rate are defined as above, and the sum of these may not be superior to 6/6 (100%).

Advantageously, A may be chosen from a substituted or unsubstituted phenyl, a substituted or unsubstituted biphenyl, a substituted or unsubstituted triphenyl, a substituted or unsubstituted terphenyl, a substituted or unsubstituted diphenyl ether, a substituted or unsubstituted naphthalene, a substituted or unsubstituted phenanthrene, a substituted or unsubstituted anthracene and a substituted or unsubstituted pyridinyl, preferably a substituted or unsubstituted phenyl. A may be substituted by at least one group chosen from a halo group (preferably F, Br or I), $-R^4$ in which $R^4$ is a $C_1$ to $C_4$ alkyl chain (preferably -Me), $-OR^4$ in which $R^4$ is a $C_1$ to $C_4$ alkyl chain (preferably -OMe), -OH, $-NO_2$ and $-NH_2$. Preferably, A may be chosen from the linkers shown in the table 1 below:

| Table 1: Structures of A | 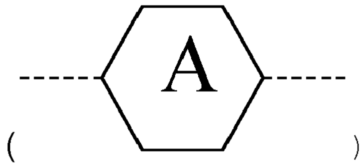 |
|---|---|
| 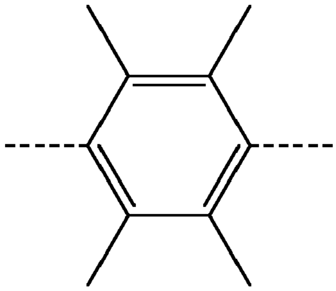 | 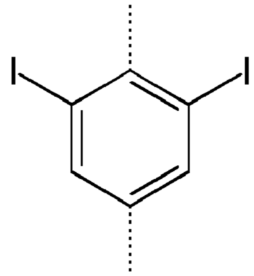 |
| 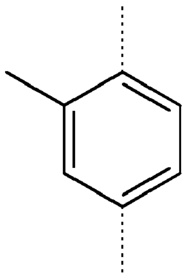 | 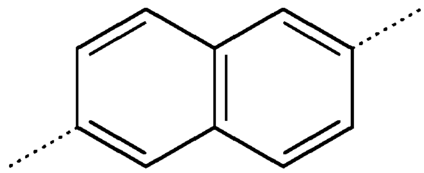 |

| | |
|---|---|
| 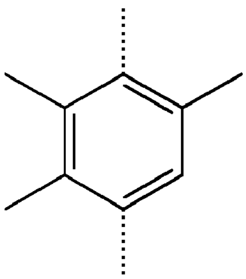 | 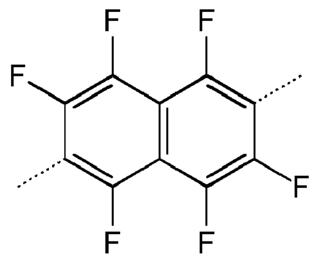 |
| 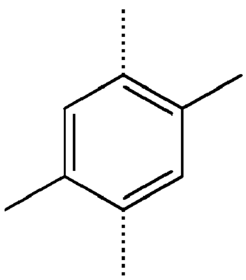 | 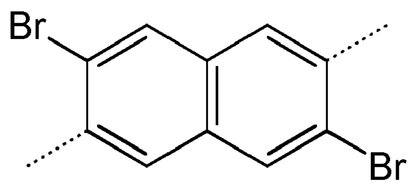 |
| 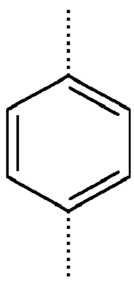 | 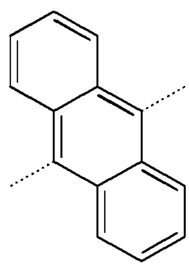 |

(continued)

| | |
|---|---|
| 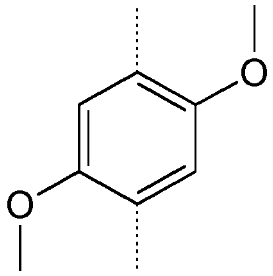 | 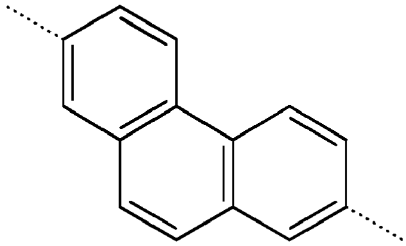 |
| 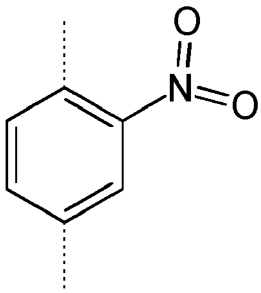 | 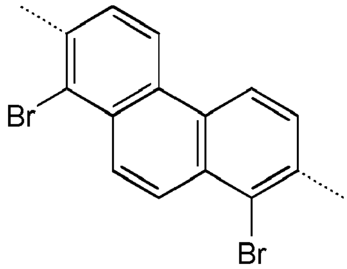 |

| | |
|---|---|
| 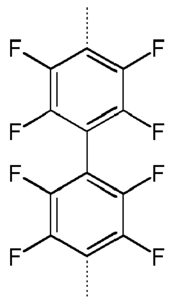 | 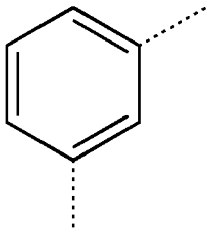 |
| 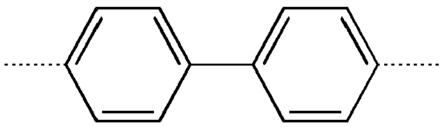 | 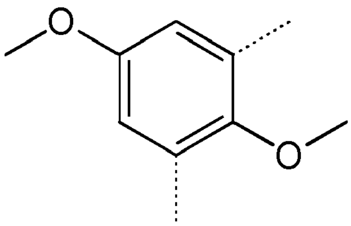 |
| 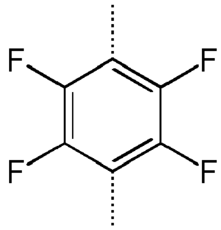 | 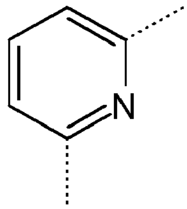 |

(continued)

| | |
|---|---|
| 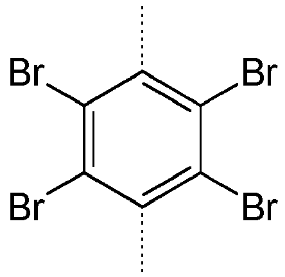 | 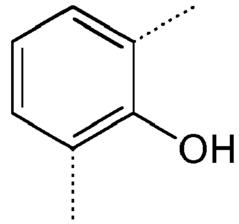 |
| 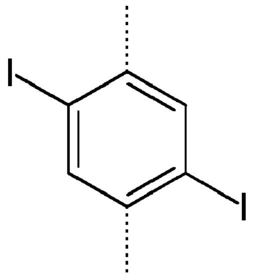 | 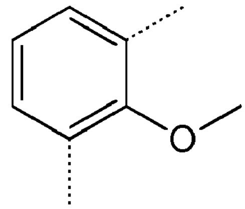 |

| | |
|---|---|
| 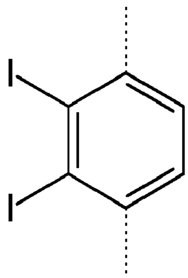 | 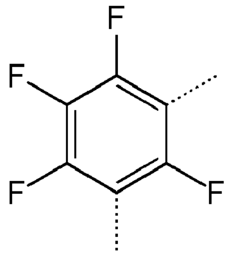 |
| | 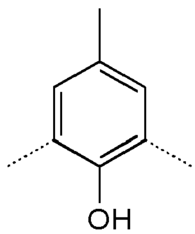 |

Advantageously, each $Y^1$ may independently represent a bond or forms, together with the nitrogen of the trianglamine, a functional group chosen from urea (-N-C(O)-NH-), carbamate (-N-C(O)-O-) and amide (-N-C(O)-). When $Y^1$ is a bond, the functional group connecting the substituent or linker to the trianglamine is tertiary amine.

Advantageously, $R^1$ may independently represent a mono- or bifunctional radical chosen from H, a linear or cyclic, optionally branched, saturated or unsaturated, optionally substituted $C_1$ to $C_{30}$ hydrocarbon chain optionally comprising a heteroatom such as O or N, an optionally substituted $C_1$ to $C_{20}$ alkylaryl, an optionally substituted $C_1$ to $C_{20}$ alkylheteroaryl group, a substituted or unsubstituted $C_4$ to $C_6$-membered aryl or heteroaryl group or a polyether comprising from 2 to 100 monomers (for example PEG, PPG or PEG-PPG copolymer). $R^1$ may preferably be H or a $C_2$ to $C_{22}$ linear or cyclic alkyl or heteroalkyl chain, more preferably H or a $C_4$ to $C_{12}$ linear alkyl chain, and even more preferably chosen from H, butyl, hexyl, octyl and dodecyl. $R^1$ may be H, an optionally substituted $C_1$ to $C_{20}$ alkylaryl or an optionally substituted $C_1$ to $C_{20}$ alkylheteroaryl group, preferably chosen from H, benzyl, methoxybenzyl and n-butylphenyl. $R^1$ may be chosen from H, a substituted or unsubstituted $C_4$ to $C_6$-membered aryl or heteroaryl linker, and a polyether comprising from 2 to 100 monomers (preferably PEG, PPG or PEG-PPG copolymer), preferably a methyl substituted phenyl. Preferably, $R^1$ is H, a $C_2$ to $C_{14}$ linear alkyl chain or an optionally substituted $C_1$ to $C_{20}$ alkylaryl or an optionally substituted $C_1$ to $C_{20}$ alkylheteroaryl group, when a = 1 and b = c = 0. Preferably, $R^1$ is H, a substituted or unsubstituted $C_4$ to $C_6$-membered aryl or heteroaryl linker or a polyether when a = 1 and b = 0 or 1. $R^1$ may be substituted

by a $C_1$ to $C_3$ alkoxy, a halo group, a hydroxyl group or a acyl group (preferably $C(O)CH_3$).

Advantageously, $Y^2$ may independently represent a functional group chosen from hydroxyl (-OH), ester (-C(O)O- or -O-C(O)-), diester (-O-C(O)-$R^5$-C(O)-O-, in which $R^5$ is a $C_1$ to $C_8$ alkyl chain), carbamate (-NH-C(O)-O- or -O-C(O)-NH-), ether (-O-), amine (-NH- or -NR- in which R is a protecting group such as Boc), acrylate ($CH_2$=CHC(O)O-), methacrylate ($CH_2$=C($CH_3$)C(O)O-), -Si$OR^4_3$ in which $R^4$ is a $C_1$ to $C_4$ alkyl chain, halo group, a maleinimido group, a silicagel and a resin such as polystyrene resin. Preferably, $Y^2$ independently represents a functional group chosen from hydroxyl (-OH), ester (-C(O)O- or -O-C(O)-), diester (-O-C(O)-$R^5$-C(O)-O-, in which $R^5$ is a $C_1$ to $C_8$ alkyl chain), carbamate (-NH-C(O)-O- or -O-C(O)-NH-), ether (-O-), amine (-NH- or -NR- in which R is a protecting group such as Boc), acrylate ($CH_2$=CHC(O)O-), methacrylate ($CH_2$=C($CH_3$)C(O)O-), - Si$OR^4_3$ in which $R^4$ is a $C_1$ to $C_4$ alkyl chain, halo group and a maleinimido group. More preferably, $Y^2$ is chosen from hydroxyl (-OH), acrylate ($CH_2$=CHC(O)O-), methacrylate ($CH_2$=C($CH_3$)C(O)O-), -Si$OR^4_3$ when a = b = 1 and c = 0. Preferably, $Y^2$ is chosen from ester (-C(O)O- or -O-C(O)-), diester (-O-C(O)-$R^5$-C(O)-O-, carbamate (-NH-C(O)-O- or -O-C(O)-NH-), or ether (-O-) when a = b = 1 and c = 1.

Advantageously, $R^3$ may represent a mono-, bi-, tri- or tetrafuntional radical comprising from 1 to 500 carbon atoms, optionally comprising hetero atoms such as O, N, Si or halo (e.g F, Br I). Preferably, $R^3$ may be chosen from an optionally substituted (e.g. -OH, F) linear or branched, saturated or unsaturated, $C_1$ to $C_{22}$ hydrocarbon chain, an optionally substituted $C_1$ to $C_{20}$ alkylaryl, an optionally substituted $C_1$ to $C_{20}$ alkylheteroaryl linker, a substituted or unsubstituted $C_4$ to $C_{12}$-membered aryl or heteroaryl linker, a polyacrylate radical comprising from 2 to 100 acrylate monomers, a polymethacrylate radical comprising from 2 to 100 methacrylate monomers, a polyether radical comprising from 2 to 200 ether monomers (i.e. PEG, PPG or PEG-PPG copolymer), and a copolymer having acrylate, methacrylate and/or ethylene moieties and comprising from 2 to 200 monomers. Preferably, $R^3$ is a hydrocarbon chain, an alkylaryl, an alkylheteroaryl, or a heteroaryl linker when a = 0, b = 0 c = 1 and d = 1, 2 or 3. Preferably, $R^3$ is not a cycloalkyl or an aryl when a = b = 0, c = 1 and d is different from 0.

Advantageously, the compound of formula I may be chosen from the compounds wherein the substituent (or linker, when d is different from 0) of formula

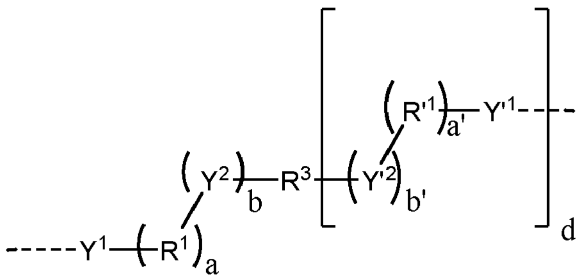

is chosen from the following structures:

Table 2 : Examples of structure of substituent or linker. In left column, the dash line indicates the point(s) of attachment to $Y^1$ and $Y'^1$ or to the N of trianglamine (when $Y^1$ and/or $Y'^1$ are bonds).

| Structure of substituent or linker (not showing $Y^1$ and $Y'^1$) | $R^1$ | $Y^1$ | other |
|---|---|---|---|
| 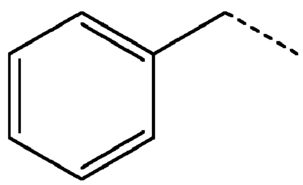 | benzyl | Urea | a = 1<br>b = 0<br>c = 0 |
| 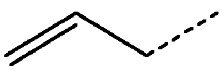 | propenyl | Urea | a = 1<br>b = 0<br>c = 0 |

(continued)

| Structure of substituent or linker (not showing $Y^1$ and $Y'^1$) | $R^1$ | $Y^1$ | other |
|---|---|---|---|
| 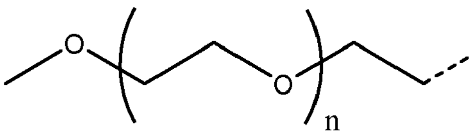 in which n is from 2 to 100 | polyether | Urea | a= 1<br>b = 1<br>c = 1<br>d = 0<br>$Y^2$-$R^3$ = methoxy |
| 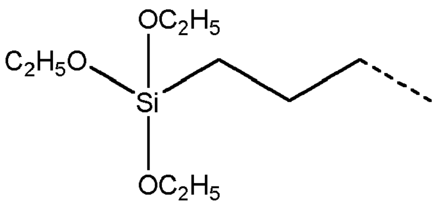 | Substituted alkyl | Urea | a= 1<br>b = 1<br>c = 0<br>$Y^2$ = $SiOEth_3$ |
| 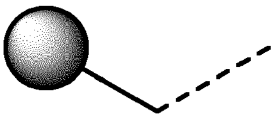 | Substituted alkyl | Urea | a= 1<br>b = 1<br>c = 0<br>$Y^2$ = polystyrene resine |
| 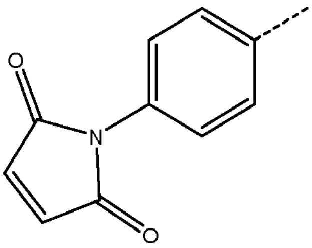 | Substituted aryl | Urea | a= 1<br>b = 1<br>c = 0<br>$Y^2$ = maleinimid o |
| 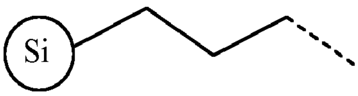 | Substituted alkyl | Urea | a= 1<br>b = 1<br>c = 0<br>$Y^2$ = silica gel |
| 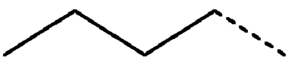 | Alkyl | Urea | a= 1, b = 0<br>et c = 0 |
| 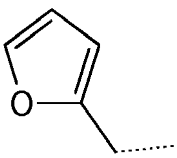 | alkylheteroar yl | Urea | a= 1<br>b = 0<br>c = 0 |
| 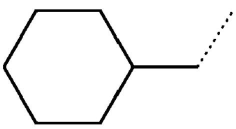 | cycloalkyl | Urea | a= 1<br>b = 0<br>c = 0 |
| 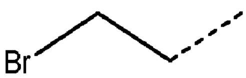 | Substituted alkyl | Urea | a= 1<br>b = 1<br>c = 0<br>$Y^2$ = Br |

(continued)

| Structure of substituent or linker (not showing $Y^1$ and $Y'^1$) | $R^1$ | $Y^1$ | other |
|---|---|---|---|
| 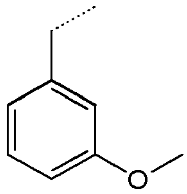 | Substituted alkylaryl | Urea | a= 1<br>b = 1<br>c = 1<br>$Y^2$-$R^3$ = methoxy |
| 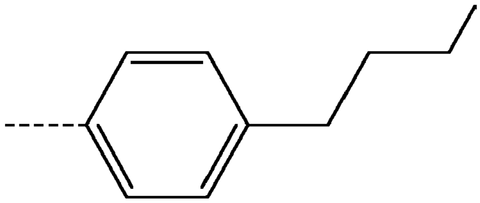 | Substituted aryl | Urea | a= 1<br>b = 0<br>c = 0 |
| 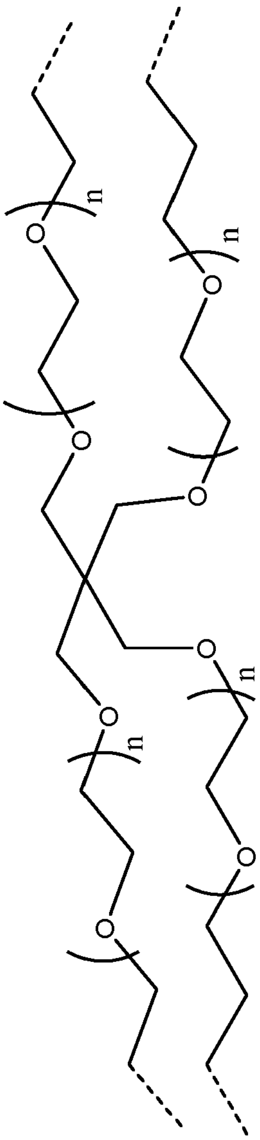<br>in which each n is from 2 to 100 | polyether | Urea | a=a'= 1<br>b=b'= 1<br>c= 1<br>d = 3<br>$Y^2$ = $Y'^2$ = ether |

(continued)

| Structure of substituent or linker (not showing $Y^1$ and $Y'^1$) | $R^1$ | $Y^1$ | other |
|---|---|---|---|
| 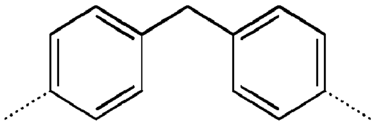 | - | Urea | a = a'= 0<br>b = b' = 0 c<br>= 1<br>d = 1<br>R3 = alkylaryl |
| 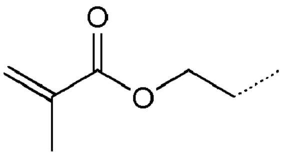 | alkyl | Urea | a=1<br>b= 1<br>c=0<br>$Y^2$ = acrylate |
| 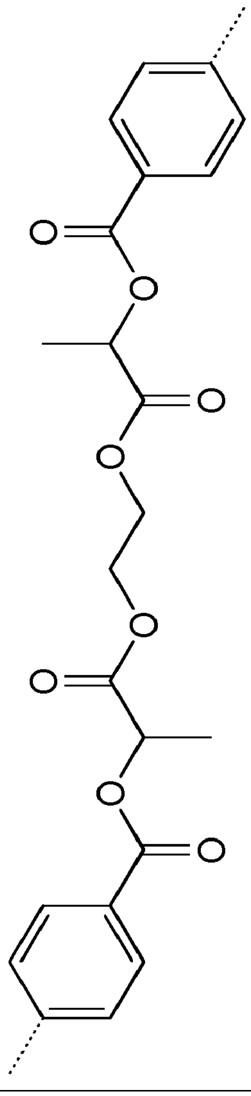 | aryl | Urea | a=1<br>b=1<br>c=1<br>d=1<br>$R^3$= copolymer<br>$Y^2$ = $Y'^2$ = ester |
| 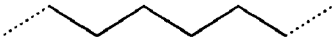 | alkyl | Urea | a = a'= 0 b = b' = 0, c = 1<br>d = 1<br>$R^3$ = alkyl |

(continued)

| Structure of substituent or linker (not showing $Y^1$ and $Y'^1$) | $R^1$ | $Y^1$ | other |
|---|---|---|---|
| 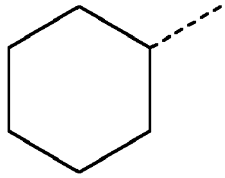 | cycloalkyl | amide | a= 1<br>b = 0<br>c = 0 |
| 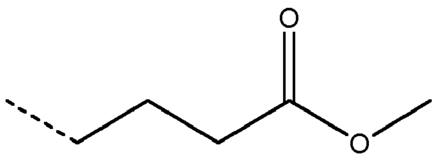 | alkyl | amide | a= 1<br>b = 1<br>c = 1<br>$Y^2$-$R^3$ = C(O)OMe |
| 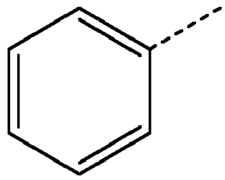 | aryl | amide | a= 1<br>b = 0<br>c = 0 |
| 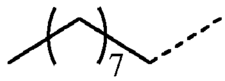 | alkyl | amide | a= 1<br>b = 0<br>c = 0 |
| 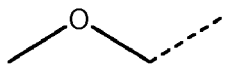 | alkyl | amide | a= 1<br>b = 1<br>c = 1<br>$Y^2$-$R^3$ = methoxy |
| 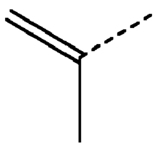 | propenyl | amide | a= 1<br>b = 0<br>c = 0 |
| 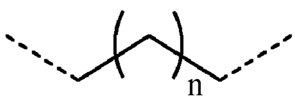<br>in which n is from 1 to 20 | - | amide | a = a'= 0<br>b = b' = 0<br><br>c = 1<br>d = 1<br>$R^3$ = alkyl |
| 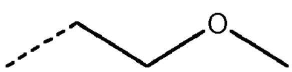 | alkyl | carbamat e | a= 1<br>b = 1<br>c = 1<br>d = 0<br>$Y^2$-$R^3$ = methoxy |
| 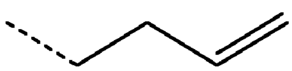 | butenyl | amide | a= 1<br>b = 0<br>c = 0 |

(continued)

| Structure of substituent or linker (not showing $Y^1$ and $Y'^1$) | $R^1$ | $Y^1$ | other |
|---|---|---|---|
| 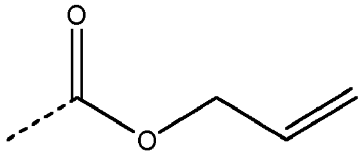 | - | amide | a = 0, b = 1<br>c= 1, $Y^2$ = ester and<br>R3 = propenyl |
| 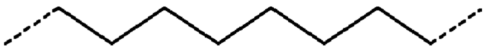 | - | amide | a = a'= 0<br>b = b' = 0<br>c = 1<br>d = 1<br>$R^3$ = alkyl |
| 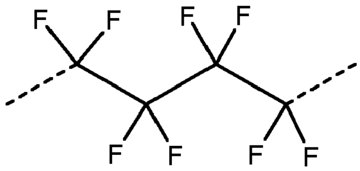 | - | amide | a = a'= 0<br>b = b' = 0<br>c = 1<br>d = 1<br>$R^3$ = fluoroalkyl |
| 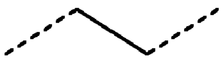 | - | amide | a = a'= 0<br>b = b' = 0<br>c = 1<br>d = 1<br>$R^3$ = alkyl |
| 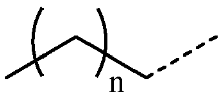<br>in which n is from 1 to 20 | alkyl | amide | a= 1<br>b = 0<br>c = 0 |
| 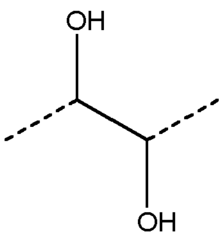 | - | amide | a = a'= 0<br>b = b' = 0<br>c = 1<br>d = 1<br>$R^3$ = di-hydroxyalk yl |
| 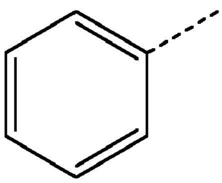 | aryl | amide | a= 1<br>b = 0<br>c = 0 |
| 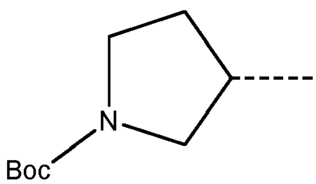 | Substituted cyclic heteroalkyl | amide | a= 1<br>b = 0<br>c = 0 |

(continued)

| Structure of substituent or linker (not showing $Y^1$ and $Y'^1$) | $R^1$ | $Y^1$ | other |
|---|---|---|---|
| 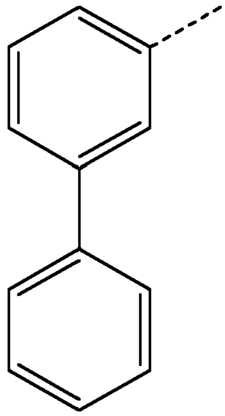 | Substituted aryl | amide | a= 1<br>b = 0<br>c = 0 |
| 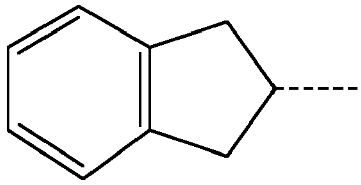 | alkylaryl | amide | a= 1<br>b = 0<br>c = 0 |
| 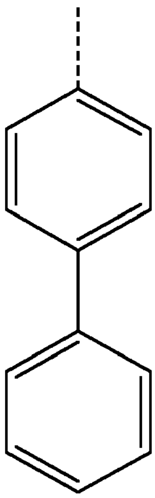 | aryl | amide | a= 1<br>b = 0<br>c = 0 |
| 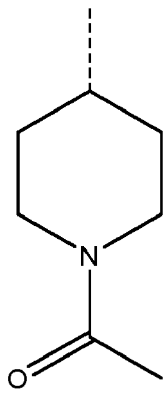 | Substituted cyclic heteroalkyl | amide | a= 1<br>b = 0<br>c = 0 |

(continued)

| Structure of substituent or linker (not showing $Y^1$ and $Y'^1$) | $R^1$ | $Y^1$ | other |
|---|---|---|---|
| 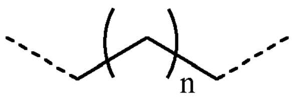<br>in which n is from 1 to 20 | - | amide | a = a'= 0<br>b = b' = 0<br>c = 1<br>d = 1<br>$R^3$ = alkyl |
| 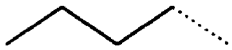 | alkyl | amine | a= 1<br>b = 0<br>c = 0 |
| 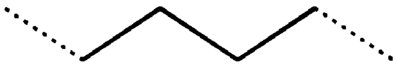 | - | amide | a = a'= 0<br>b = b' = 0<br>c = 1<br>d = 1<br>$R^3$ = alkyl |

Advantageously, the compound may be chosen from the compounds of formula I wherein the linker of formula

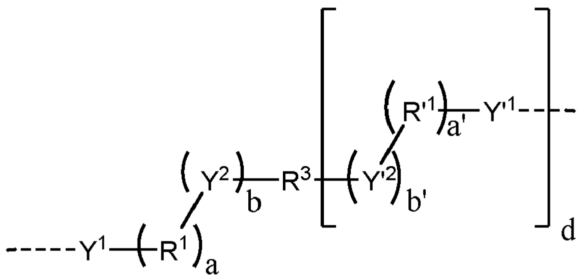

is not a di-, tri- or tetravalent phenyl, bi-phenyl, naphtyl, cyclopropyl, cyclobutyl, cyclopentyl, cyclohexyl or tetrahydrofuranyl group. Preferably, the linker of formula

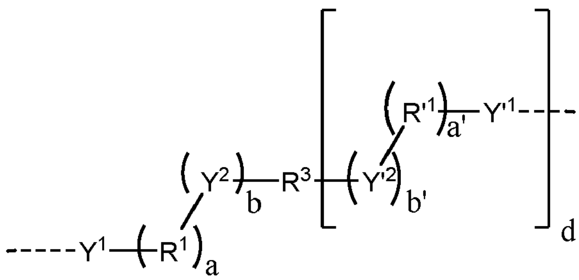

is different from the following formulas:

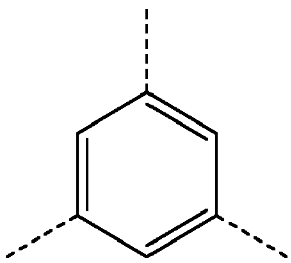 , 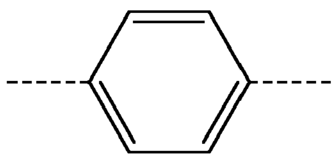 , 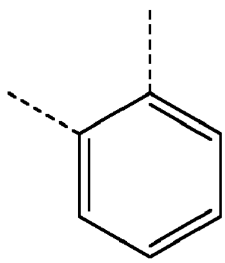 ,

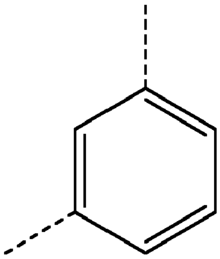 , 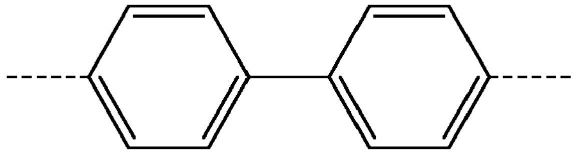

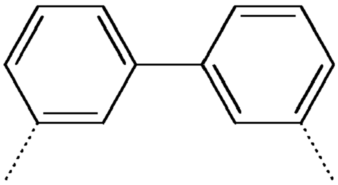 , 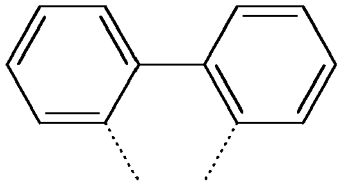

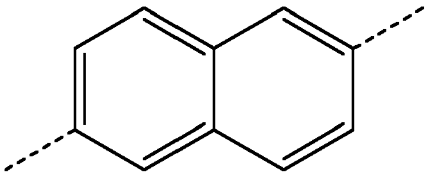 , 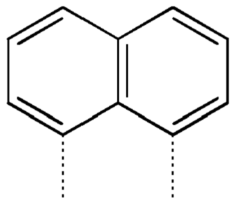 ,

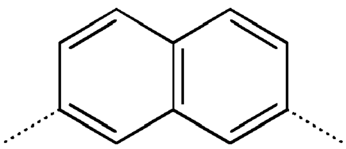 , 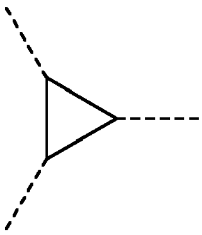 , 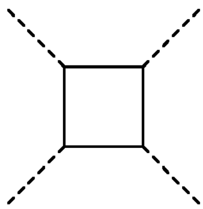 , 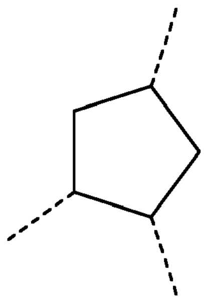 ,

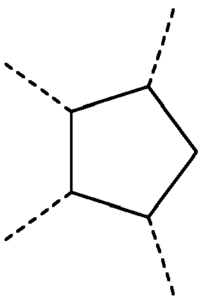 , 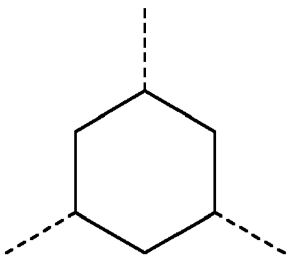 , 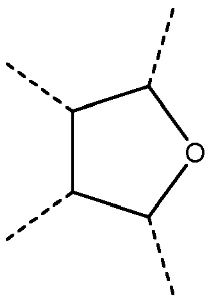 , 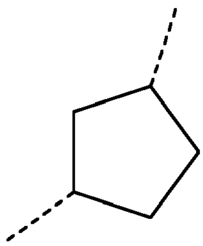 ,

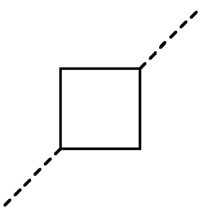 , 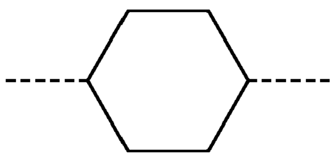 , and 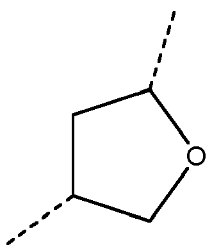 .

Advantageously, the compound according to the invention may be chosen from the following structures:

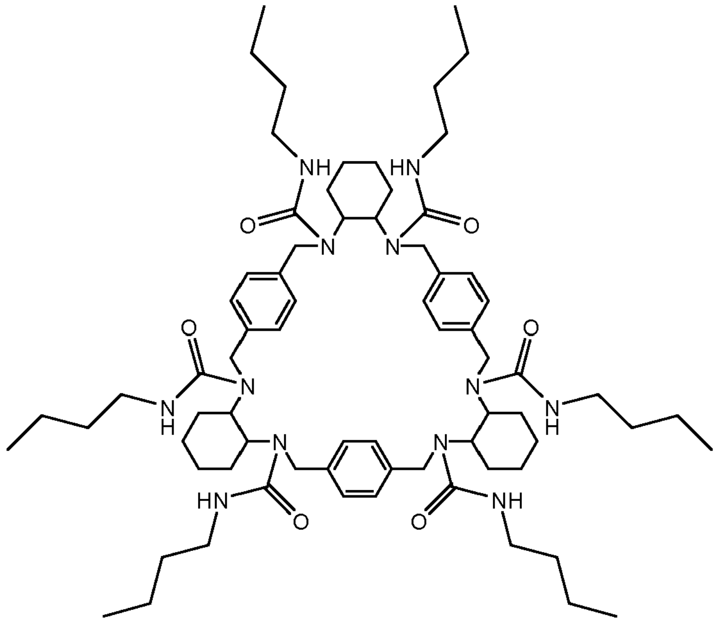

ΔC4

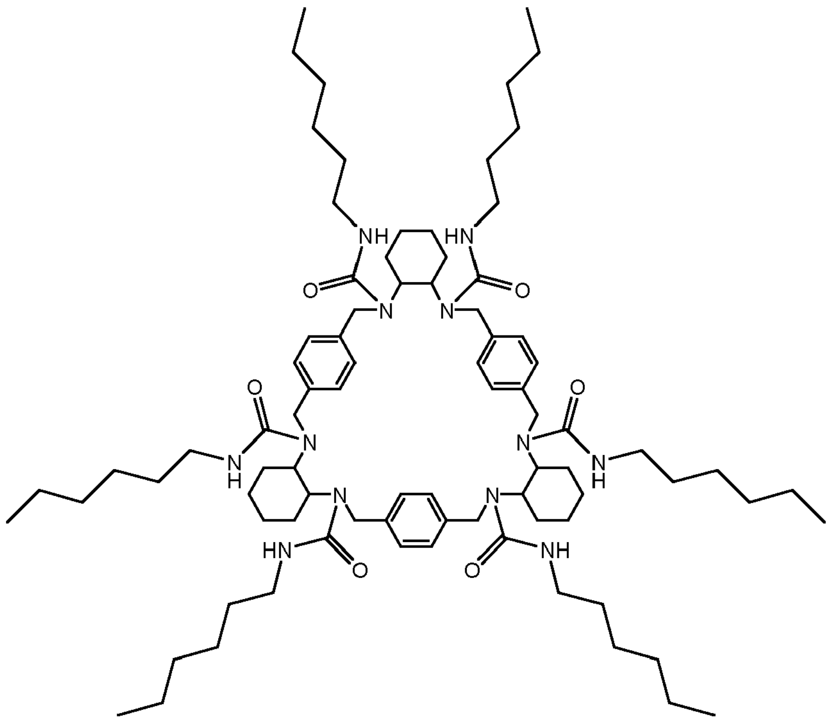

ΔC6

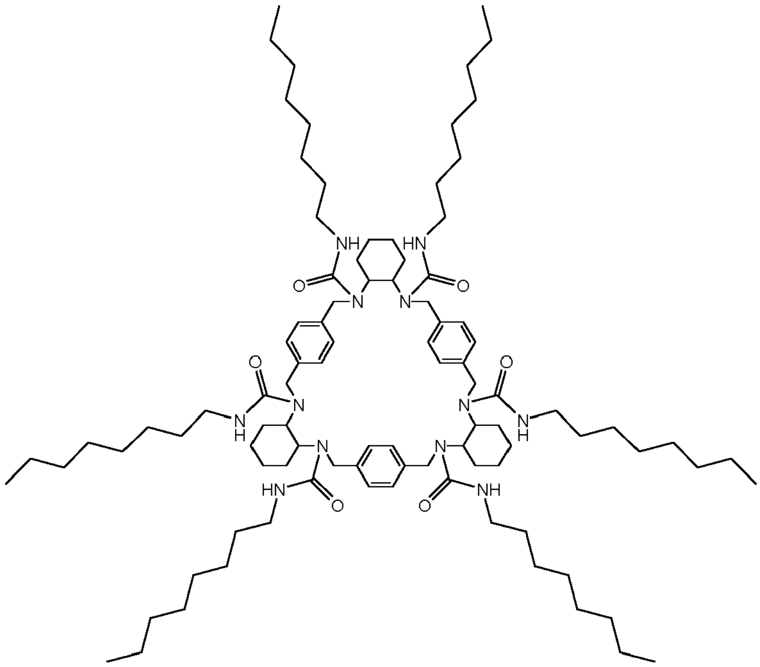

ΔC8

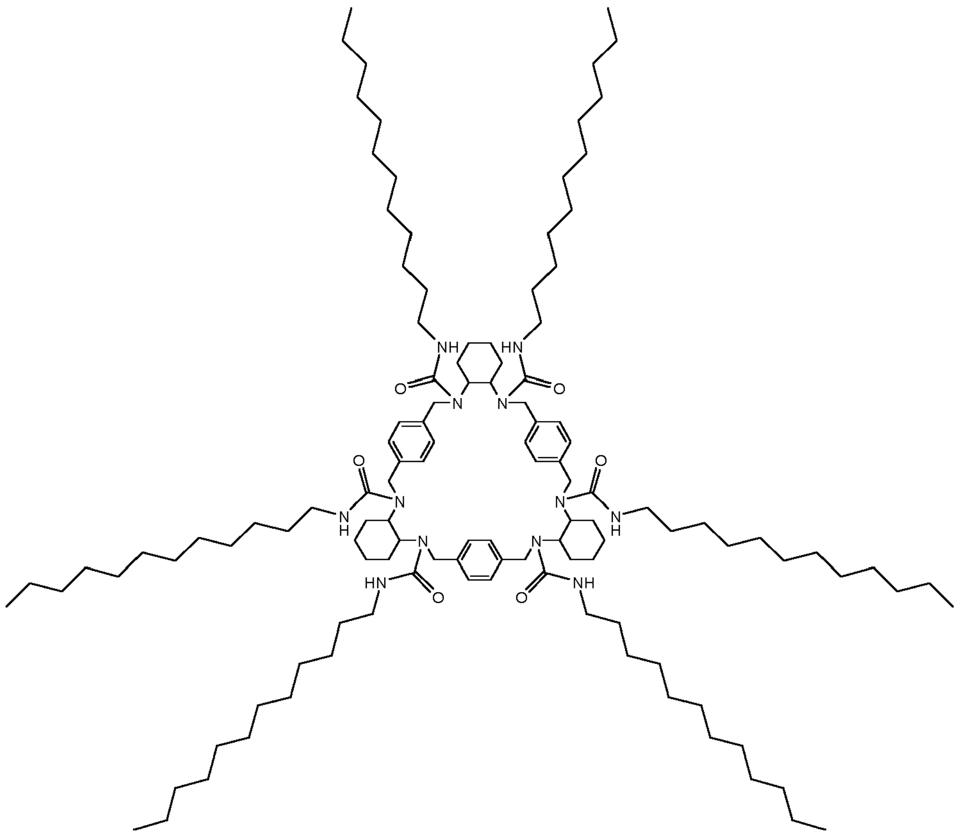

ΔC12

Advantageously, the compound according to the invention may be chosen from the following structures:

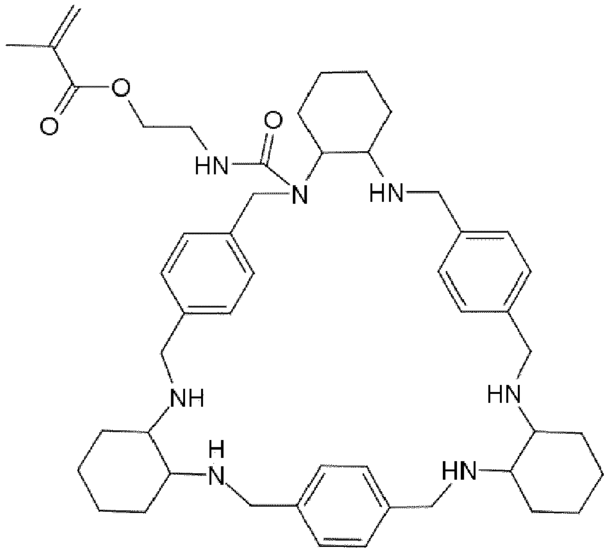

Δ@1-MMA

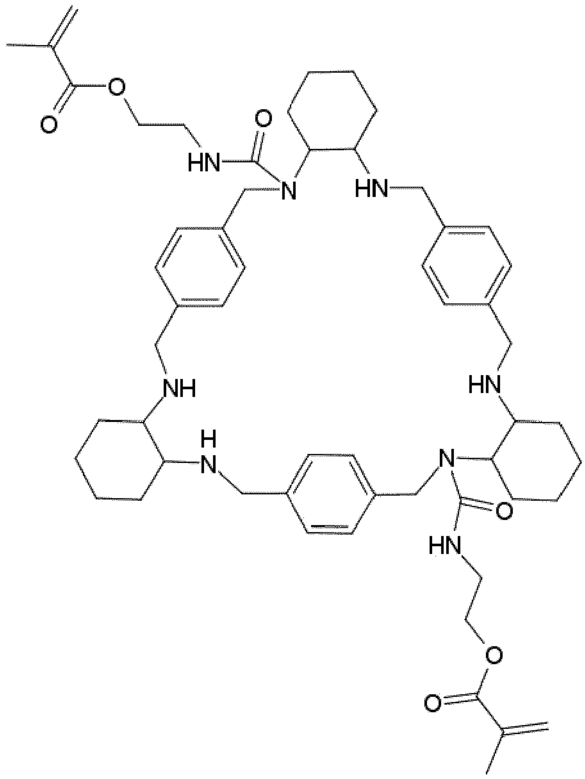

Δ@2-MMA

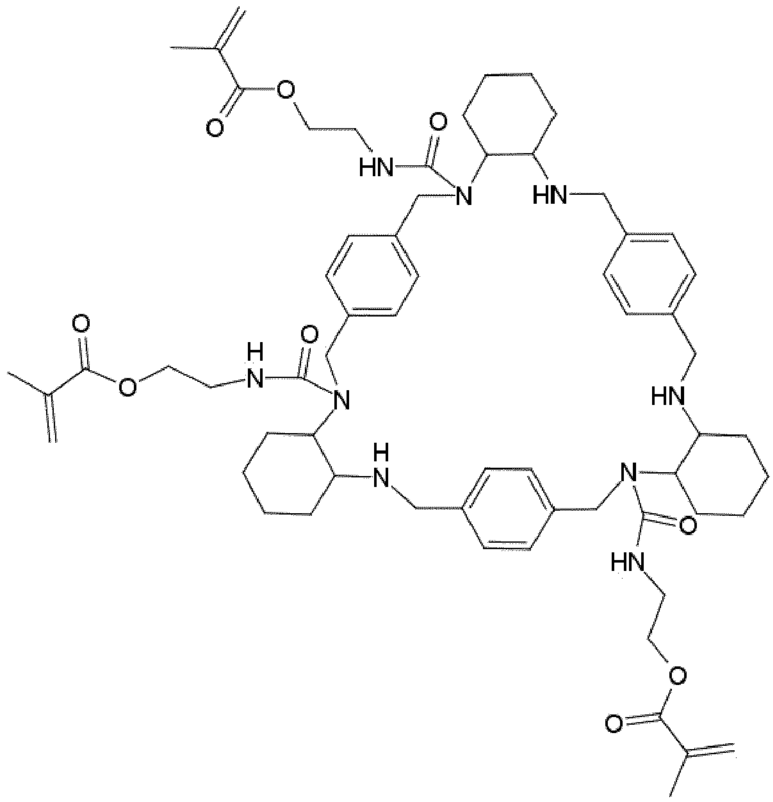

Δ@3-MMA

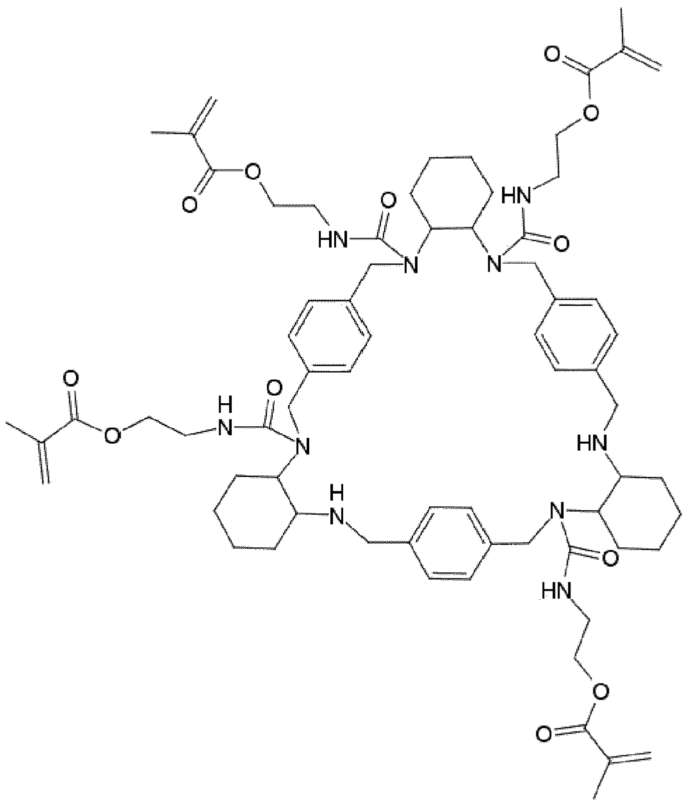

Δ@4-MMA

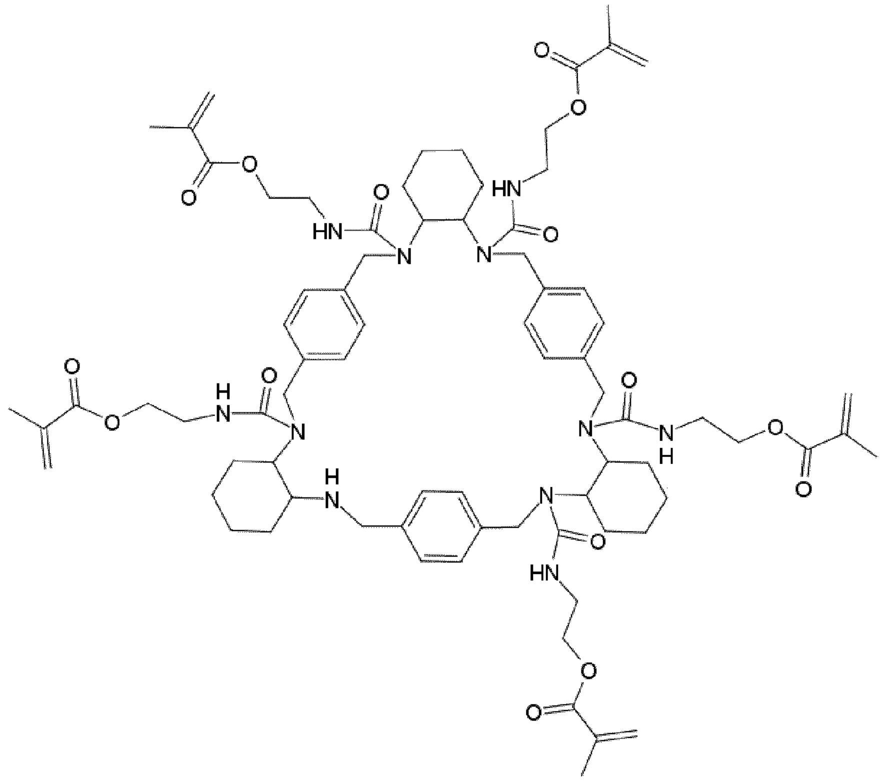

Δ@5-

MMA

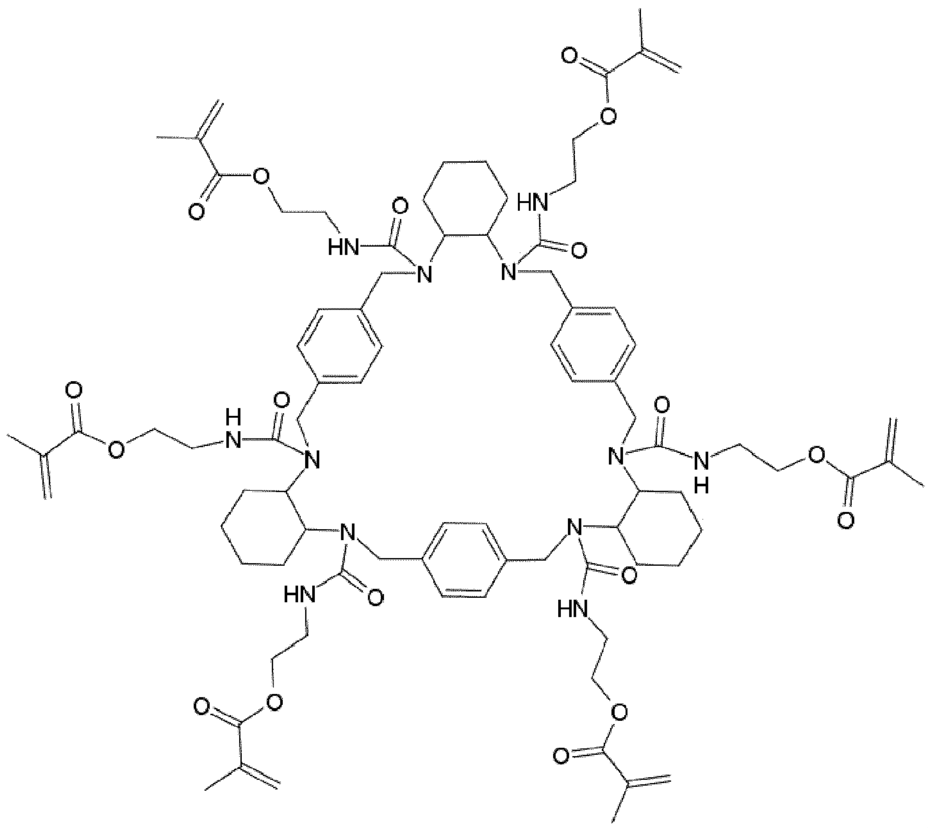

Δ@6MMA.

Advantageously, the compound according to the invention may be chosen from the following crosslinked structures:

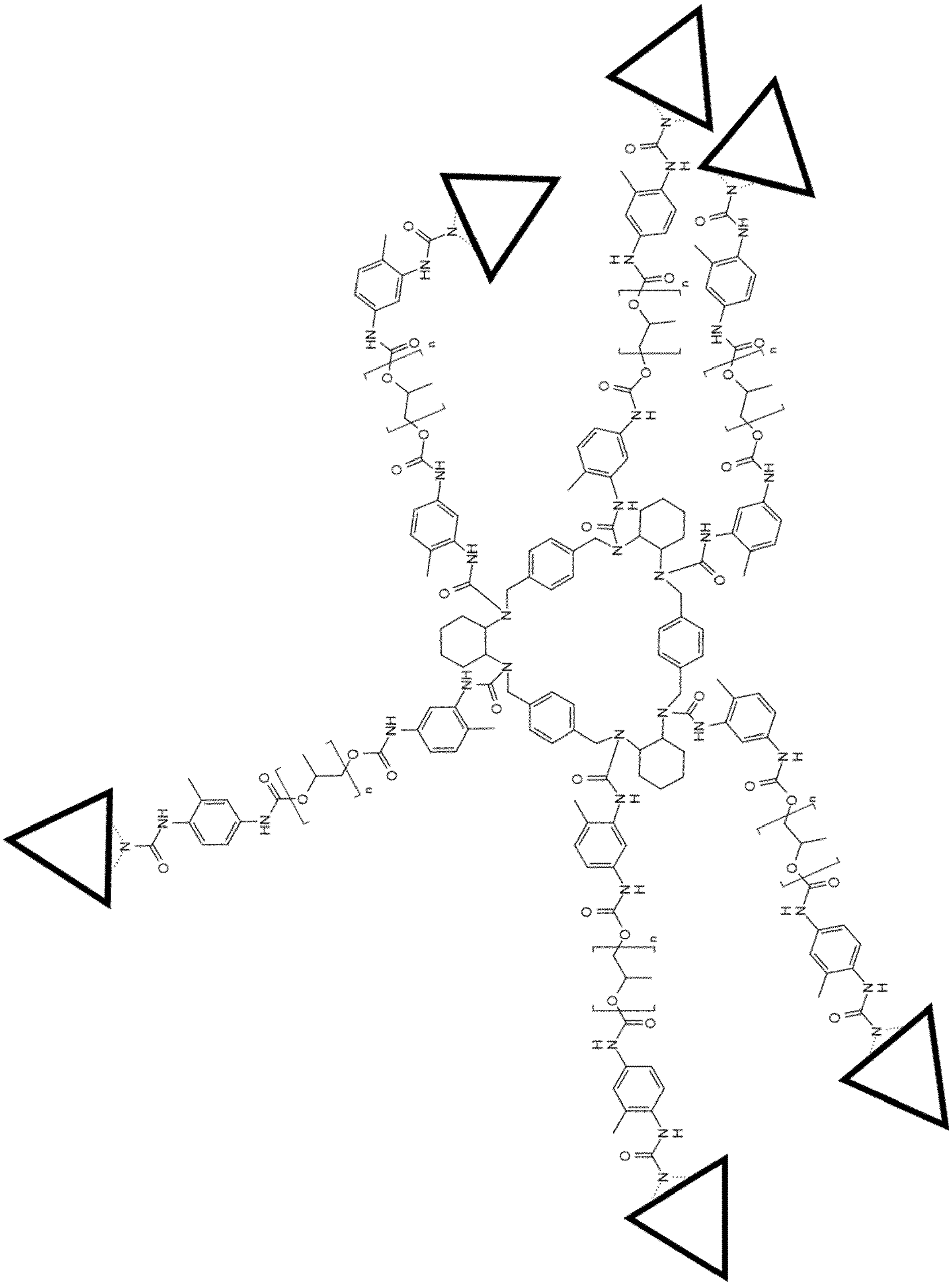

in which n is an integer from 10 to 50,

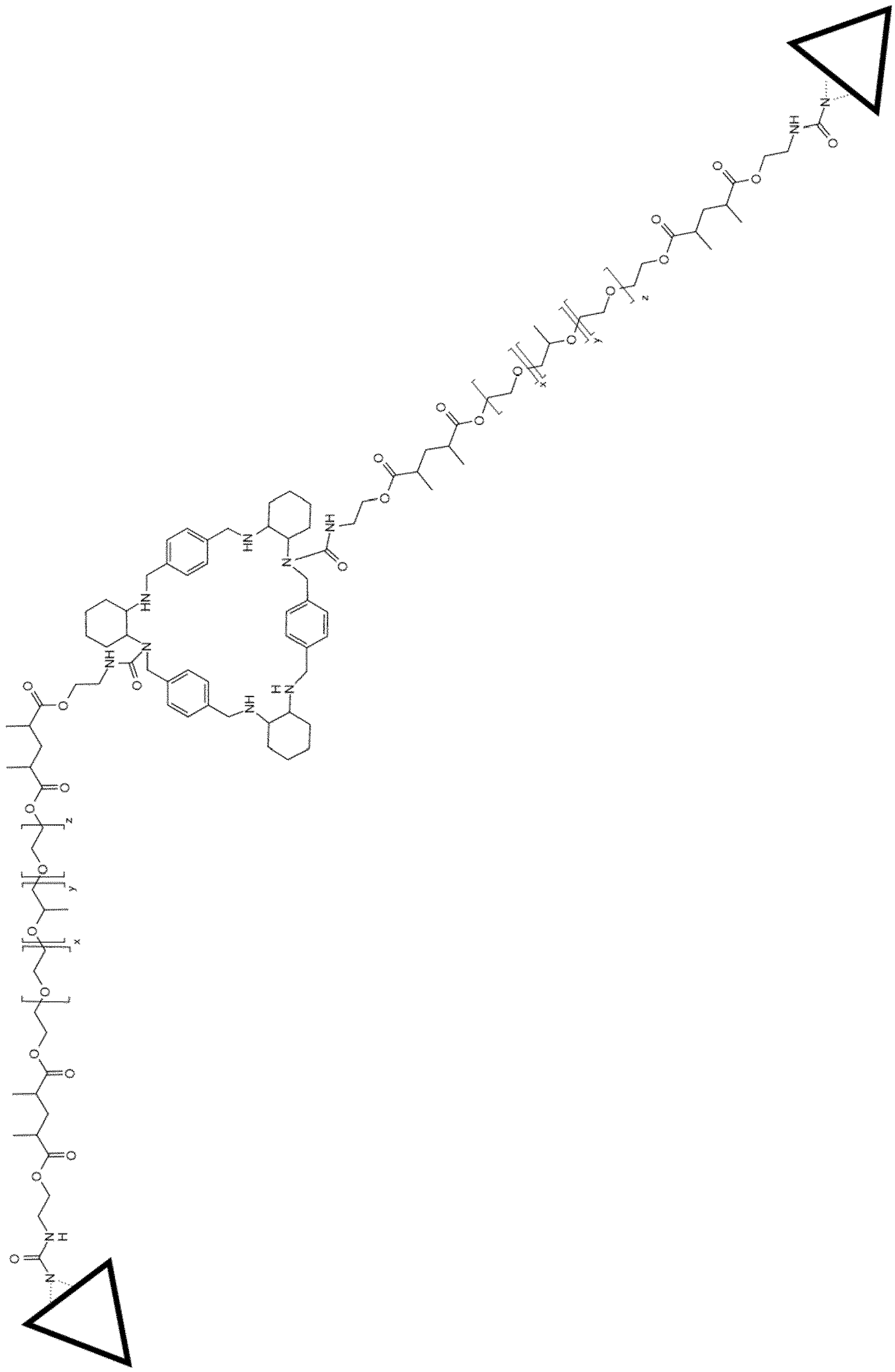

In which x is an integer from 1 to 50, y is an integer from 1 to 50 and z is an integer from 1 to 50).

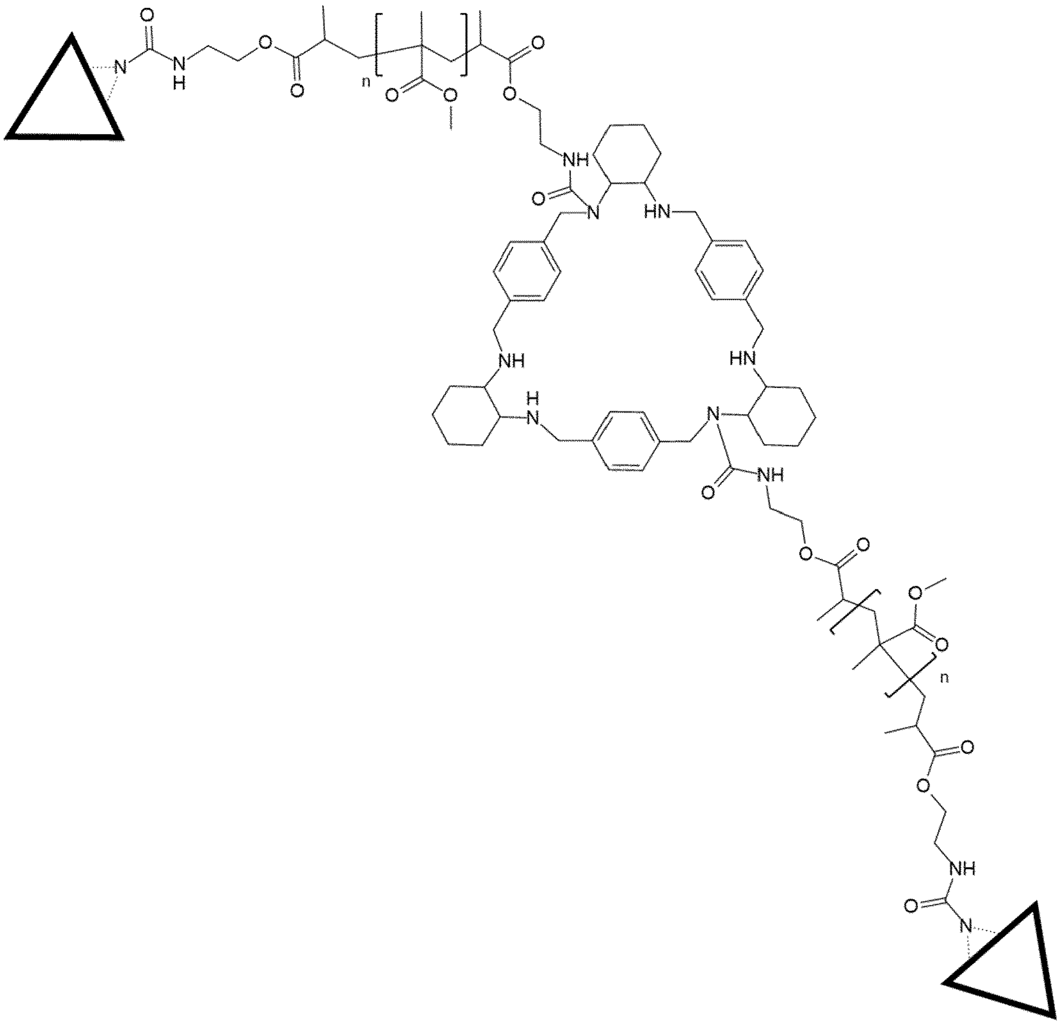

in which (n=20 to 100).

The invention also relates to A compound of formula I':

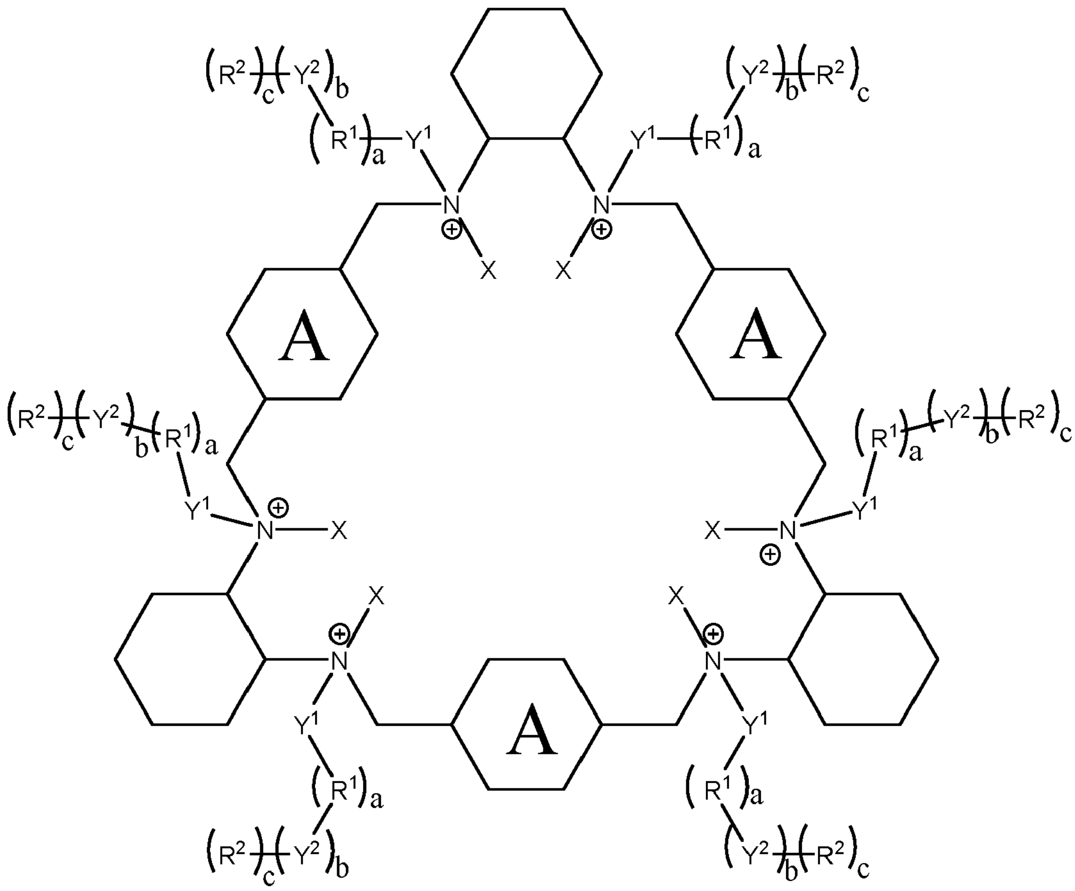

Formula I'

wherein,

- X is a halo group, preferably I, and A, $Y^1$, $R^1$, $Y^2$, $R^2$, a, b and c are defined as above.

The invention also encompasses a process of synthesis of a compound of formula I according to the invention, comprising a step of reacting a trianglamine of formula III in a solvent:

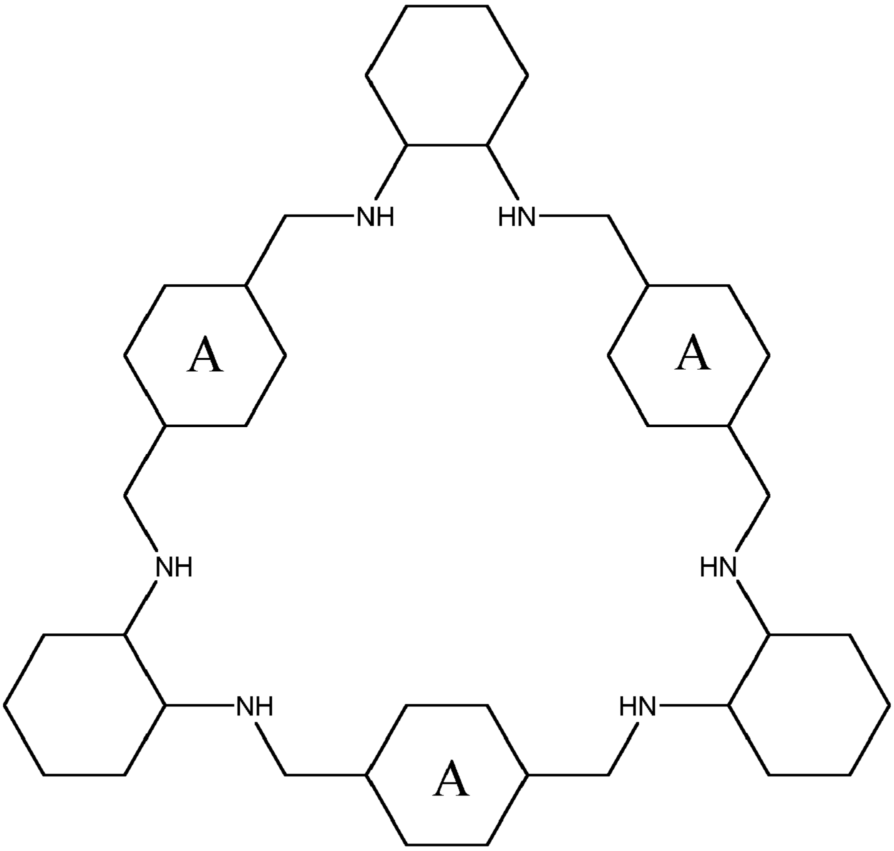

Formula III

with a compound of formula IV:

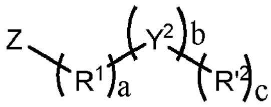

Formula IV

wherein,

- R'[2] represents a group of formula IV':

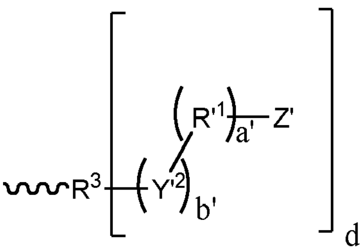

Formula IV'

- Z and Z', identical or different are chosen from isocyanate, acyle choride, acid, ester, halo and α,β-unsaturated carbonyl, and
- A, $R^1$, $R'^1$, $Y^2$, $Y'^2$, $R^3$, a, b, c, d, a' and b' are defined in previous claims.

Preferably, Z and Z' are chosen from isocyanate, acid, ester or halo and α,β-unsaturated and more preferably from isocyanate and halo.

The process may be done in a solvent. The solvent is an organic solvent chosen from dichloromethane, chloroforme, methanol, and dimethylsulfoxide. The temperature may be from 0 to 25°C.The mixture may be left to react overnight (i.e. at least 12 hours).

Advantageously, the invention relates to a process of synthesis of a trianglamine of formula III comprising the steps of:

a) reacting 1,2-diaminocyclohexane with a compound of formula V:

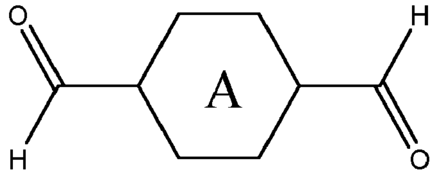

Formula V

wherein, A is defined as above,

b) reacting the compound obtained in step a) with $NaBH_4$, and obtaining the compound of formula III.

Advantageously, steps a) and b) may be done in an organic solvent. The organic solvent may be chosen from methanol, dichloromethane, chloroforme, and dimethylsulfoxide. The the temperature may be from 0 to 25°C. The mixture may be left to react overnight (i.e. at least 12 hours).

Advantageously, the process may comprise the steps:

a) reacting 1,2-diaminocyclohexane with a compound of formula V,
b) reacting the compound obtained in step a) with $NaBH_4$,
c) reacting the compound obtained in step b) with a compound of formula IV,

and obtaining the compound of formula I.

Advantageously, the compound of formula IV may be chosen from the following compounds:

Table 3 : Examples of structure of compound of formula IV.

| ***Compound Name*** | ***Structure*** |
|---|---|
| Benzyl isocyanate | 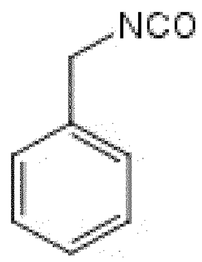 |
| Ally isocyanate | 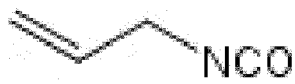 |
| mPEG5K-isocyanate | 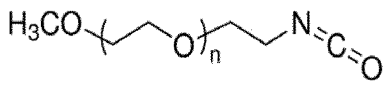 in which n is from 2 to 100 |

(continued)

| *Compound Name* | *Structure* |
|---|---|
| 3-(triethoxysilil)propyl isocyanate | 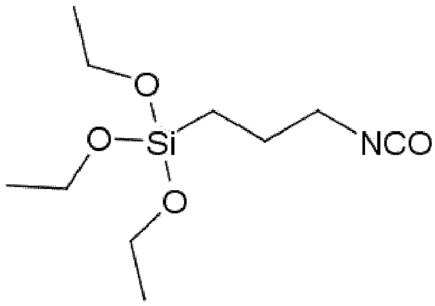 |
| polystyrene resin isocyanate | 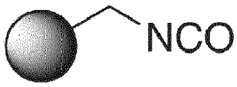 |
| 4-(Maleinimido)phenyl isocyanate | 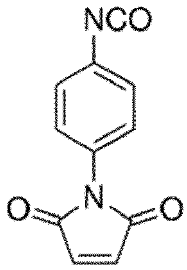 |
| 3-(Isocyanato)propyl-functionalized silica gel | 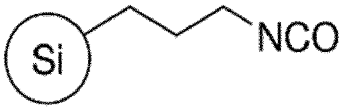 |
| Butyl isocyanate (and any other alkyl chain) | $CH_3CH_2CH_2CH_2NCO$ |
| Furfuryl isocyanate | 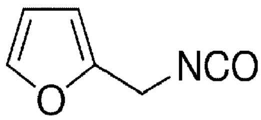 |
| Cyclohexanemethyl isocyanate | 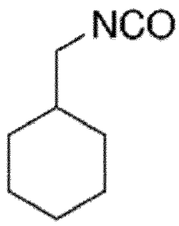 |
| 2-Bromoethyl isocyanate | 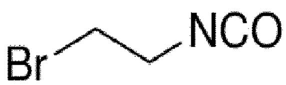 |
| 3-Methoxybenzyl isocyanate | 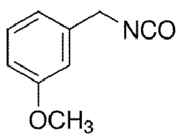 |
| 4-Butylphenyl isocyanate | 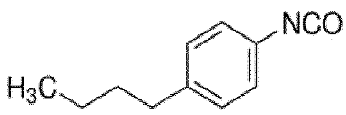 |

(continued)

| Compound Name | Structure |
|---|---|
| 4arm-PEG20K-Isocyanate | 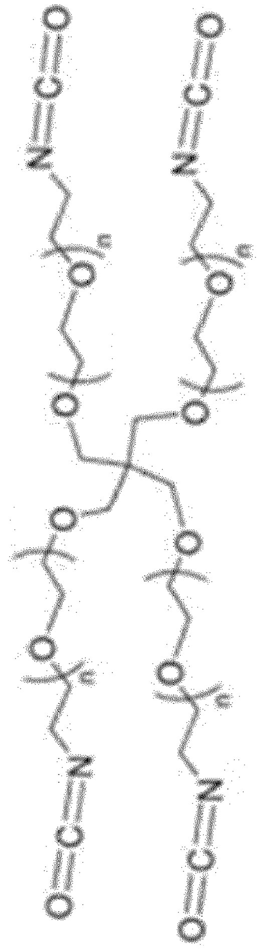 in which n is from 2 to 100 |
| 4,4'-Methylenebis(phenyl isocyanate) | 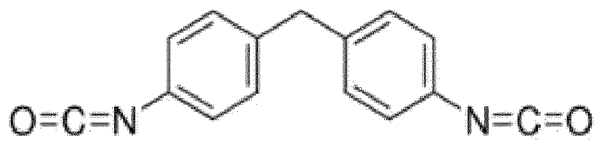 |
| 2-Isocyanatoethyl methacrylate | 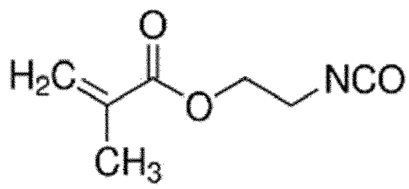 |
| Benzoic acid, 4-isocyanato-ethylene glycol bis lactic acid ester | 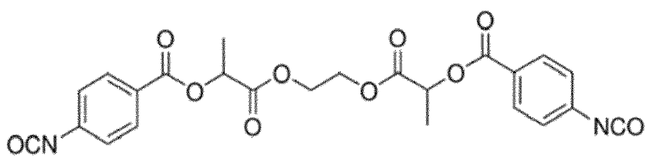 |
| Hexamethylene diisocyanate | 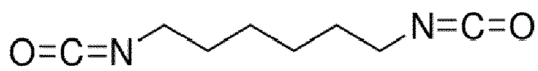 |

(continued)

| ***Compound Name*** | ***Structure*** |
|---|---|
| Cyclohexanecarboxyli c acid chloride | 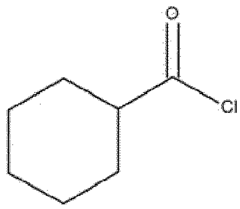 |
| Glutaric acid monomethyl ester chloride | 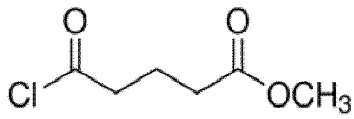 |
| Benzoyl chloride | 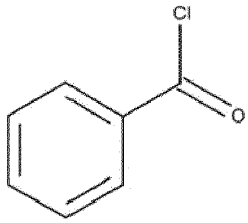 |
| Decanoyl chloride (and any length of alkyl chain) | 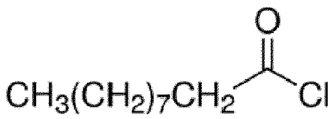 |
| Methoxyacetyl chloride | 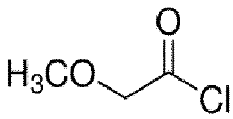 |
| Methacryloyl chloride (and the acrylate version) | 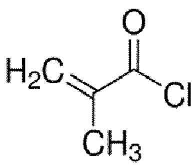 |
| Suberoyl chloride (and any length of alkyl chain) | 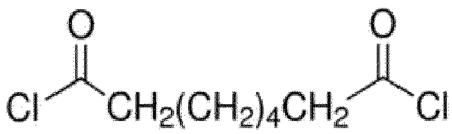 |
| 2-Methoxyethyl chloroformate (and other oxyethyl length) | 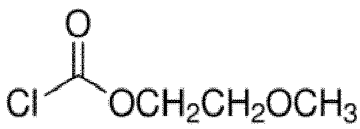 |
| 4-Pentenoyl chloride | 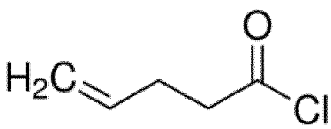 |

(continued)

| Compound Name | Structure |
|---|---|
| Allyl oxalyl chloride | 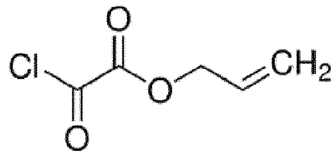 |
| Sebacoyl chloride (and any other alkyl chain length) | 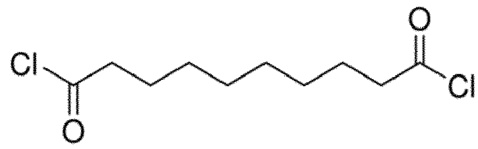 |
| Octafluoroadipoyl chloride | 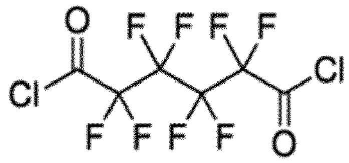 |
| Succinic acid | 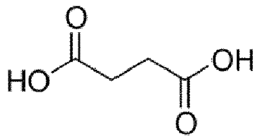 |
| Palmitic acid (and any other alkyl chain length) | 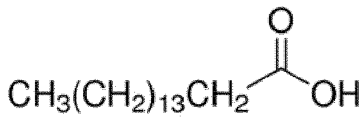 |
| Tartaric acid | 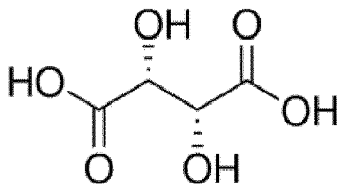 |
| Benzoic Acid | 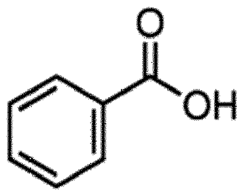 |
| N-Boc-pyrrolidine-3-carboxylic acid | 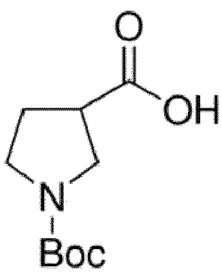 |

(continued)

| Compound Name | Structure |
|---|---|
| 4'-Methylbiphenyl-3-carboxylic acid | 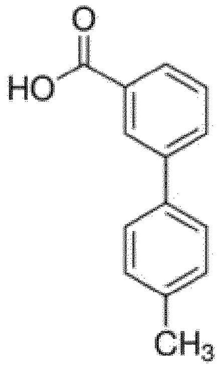 |
| Indan-2-carboxylic acid | 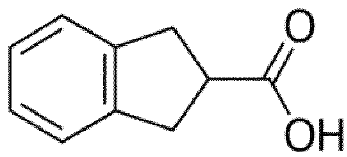 |
| Biphenyl-4-carboxylic acid | 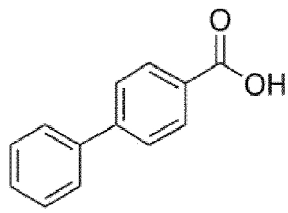 |
| 1-Acetylpiperidine-4-carboxylic acid | 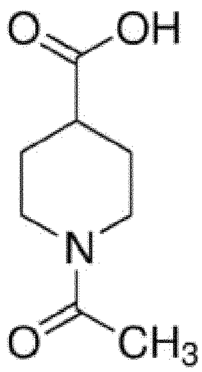 |
| Dodecanedioic acid (and any other length of alkyl chain) | 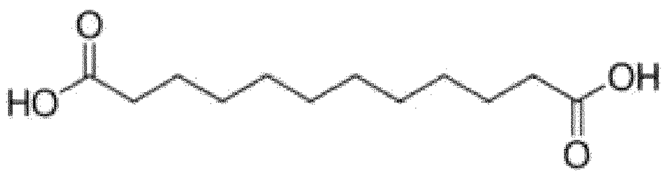 |
| Bromo or iodo butane (and any other alkyl length) | 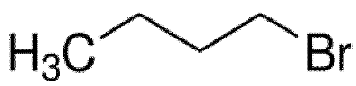 |
| | 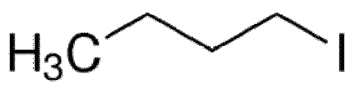 |

(continued)

| Compound Name | Structure |
|---|---|
| 1 ,4-Di bromo or iodo butane (and any other alkyl length) | 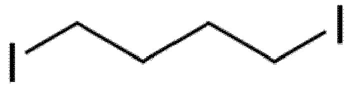 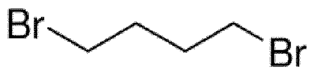 |

Advantageously, the compound of formula V may be chosen from the following compounds:

Table 4: structure of precursors of A (compounds of formula V)

| ***Names*** | ***Structures of precursor of A*** |
|---|---|
| 2,3,5,6-Tetramethylphthalaldehyde | 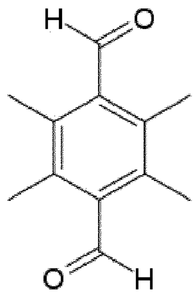 |
| 2-Methylterephthalaldehyde | 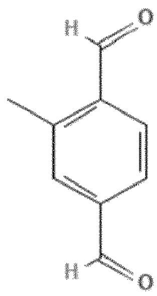 |
| 2,3,6-Trimethylphthalaldehyde | 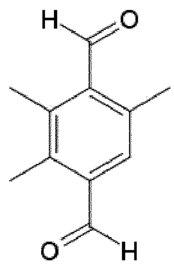 |
| 2,5-dimethylterephthalaldehyde | 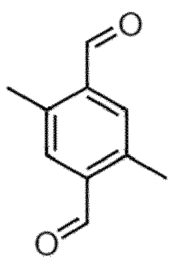 |

(continued)

| *Names* | *Structures of precursor of A* |
|---|---|
| Terephthalaldehyde | 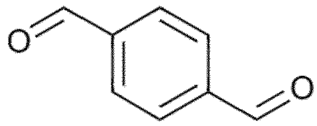 |
| 2,4-dimethoxyterephthalaldehyde | 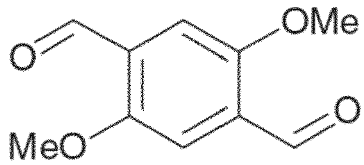 |
| 2-NitroTerephthalaldehyde | 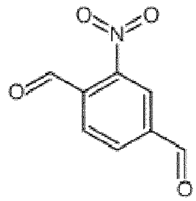 |
| 2,2',3,3',5,5',6,6'-octafluorobiphenyl-4,4'-dicarbaldehyde | 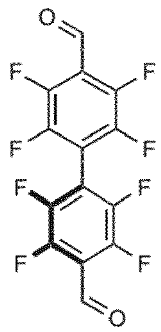 |
| Biphenyl-4,4'-dicarbaldehyde | 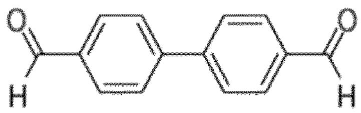 |
| 2,3,5,6-Tetrafluoroterephthalaldehyde | 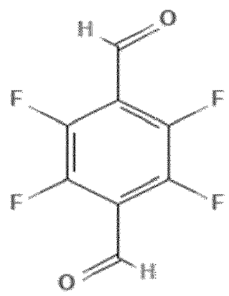 |
| 2,3,5,6-Tetrabromo-1,4-benzenedicarboxaldehyde | 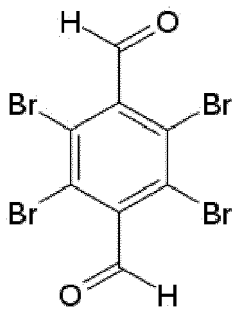 |

(continued)

| *Names* | *Structures of precursor of A* |
|---|---|
| 2,5 diiodo-1,4-benzenedicarboxaldehyde | 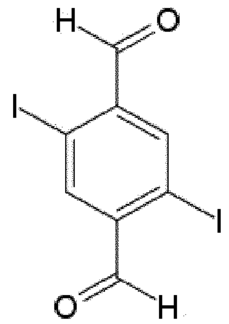 |
| 2,3 diiodo-1,4-benzenedicarboxaldehyde | 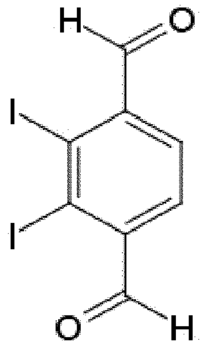 |
| 2,6 diiodo-1,4-benzenedicarboxaldehyde | 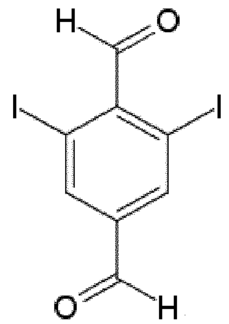 |
| 2,6-Naphthalenedicarboxaldhyde | 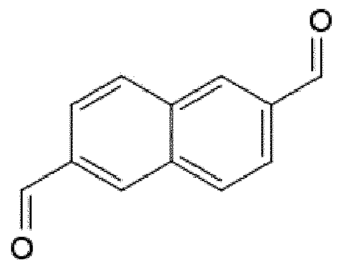 |
| 1,3,4,5,7,8-hexafluro-2,6-naphtalenedicarboxaldhyde | 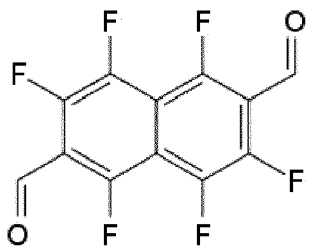 |

(continued)

| ***Names*** | ***Structures of precursor of A*** |
|---|---|
| 1,5-dibromo-2,6-naphthalenedicarboxaldhyde | 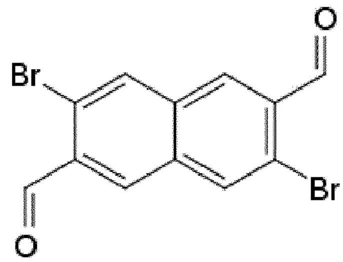 |
| 9,10-Anthracenedicarboxaldhyde | 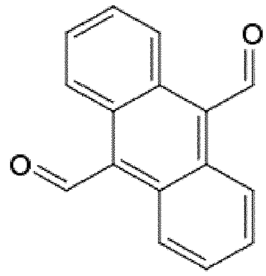 |
| 2,7-phenanthrendicarboxaldehyde | 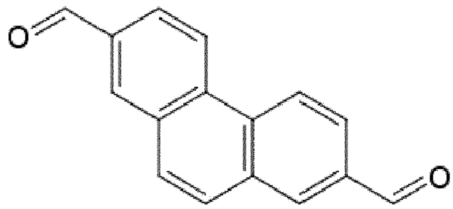 |
| 1,8-dibromo-2,7-phenanthrendicarboxaldehyde | 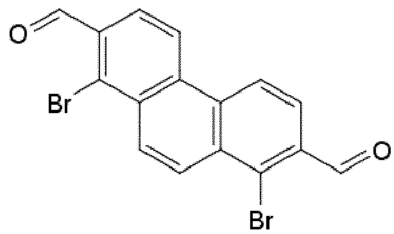 |
| 1,3-benzenedicarboxalehyde | 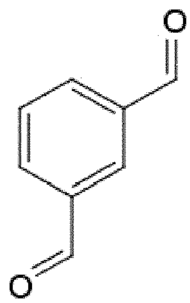 |
| 2,5-dimethoxy-1,3-benzenedicarboxalehyde | 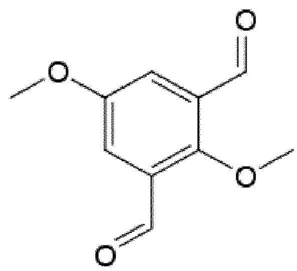 |

(continued)

| *Names* | *Structures of precursor of A* |
|---|---|
| 2,6-Pyridinedicaboxaldehyde | 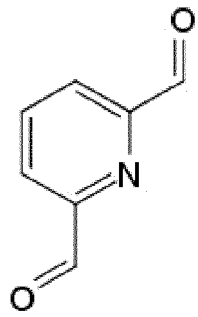 |
| 2-hydroxy-1,3-benzenedicarboxaldehyde | 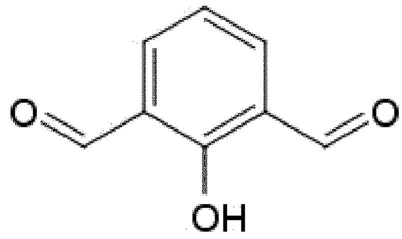 |
| 2-Methoxy-1,3-benzendicarboxaldehyde | 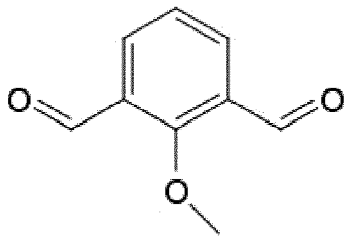 |
| 2,4,5,6-tetrfluoro-1,3-benzenedicarboxaldehyde | 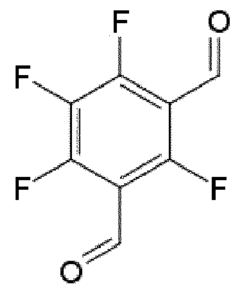 |
| 2,6-diformyl-4-methylphenol | 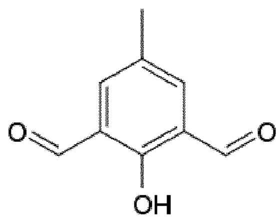 |

Advantageously, the compound of formula IV is not trimesic acid chloride, terephthalic acid chloride, isophthalic acid chloride, biphenyl dicarboxylic acid chloride, and naphthalene dicarboxylic acid dichloride cyclopropane tricarboxylic acid chloride, cyclobutane tetracarboxylic acid chloride, cyclopentane tricarboxylic acid chloride, cyclopentane tetracarboxylic acid chloride, cyclohexane tricarboxylic acid chloride, tetrahydrofuran tetracarboxylic acid chloride, cyclopentane dicarboxylic acid chloride, cyclobutane dicarboxylic acid chloride, cyclohexane dicarboxylic acid chloride, and tetrahydrofuran dicarboxylic acid chloride.

On another aspect, the invention relates to the use of a compound of formula I according to the invention, for the decontamination of air, water or sea water. Preferably, the use according to the invention is the use of a compound

of formula I according to the invention, for the decontamination of air, water or sea water from toxic material chosen from radioactive elements such as radionuclides, per- and polyfluoroalkyl substances (PFAS), dyes, metal ions, iodine, and heavy metals. The invention relates to the use of a compound of formula I according to the invention, in gas separation such as the separation of $CO_2$, $N_2$, $NH_3$, $H_2$, $O_2$ or $CH_4$. The invention relates to the use of a compound of formula I according to the invention for desalination of water. The invention also encompasses a method of production of drinkable water via filtration and desalination of sea water comprising a step of contacting water or sea water with a (crosslinked or not crosslinked) compound of formula I according to the invention.

The invention encompasses a gel (hydrogel or organogel) comprising a compound of formula I according to the invention and a solvent. The gel may comprise from 20 to 80 wt.% of solvent and from 20 to 50 wt.% of (crosslinked or not crosslinked) compound of formula I according to the invention, preferably 50:50. The solvent may be chosen from water, dimethyl sulfoxide, dichloromethane and chloroform.

In a particular embodiment of the invention, the compound of formula I mays be used as 3D ink. Preferably, the 3D ink comprise from 20 to 100 wt.% of the compound of formula I and from 20 to 100 wt.% of a solvent, preferably 50:50. Preferably, the solvent is chosen from dimethyl sulfoxide, dimethyl formamide, or dimethyl acetamide. In a preferred embodiment, the 3D ink comprising compounds of formula I, wherein $Y^2$ is acrylate or methacrylate, a = 1, b = 1 and c = 0. The 3D printing is performed by reacting the acrylate or methacrylate functional group.

Further, the invention also encompasses a membrane comprising a crosslinked compound of formula I.

**Brief description of the figures**

Figure 1 represents the UV-vis spectra of $I_2$ aqueous solution (30 mmol. $L^{-1}$) record the kinetic of $I_2$ adsorption during time after adding the gel as a function of time.

Figure 2 represents $I_2$ adsorption capacity of gels in an aqueous solution of 30 mM as a function of time.

Figure 3 represents a histogram of the adsorption efficiency (%) and cycle numbers.

Figure 4 represents the maximum uptake in high concentration of iodine in a) water and b) seawater.

Figure 5 represents the study of the adsorption of iodine in the vapor phase a) the kinetic of adsorption b) the maximum iodine uptake.

Figure 6 represents a) The kinetic of adsorption of PFAs during the time (48hours) and b) Efficiency of adsorption of PFAs.

**EXAMPLES**

**Example 1: Preparation of substituted trianglamines**

***Materials & methods***

All reagents were purchased from commercial suppliers and used without further purification. (±)-trans-1,2-diaminocyclohexane, terephthalaldehyde, triethylamine, sodium borohydride, butyl isocyanate, hexyl isocyanate, octyl isocyanate, dodecyl isocyanate were purchased from Sigma Aldrich (Saint-Quentin-Fallavier, France).

***$^1H$ NMR and $^{13}C$ NMR*** spectrum were recorded on Bruker Avance III 400 MHz and 500 MHz. NMR spectrometer in DMSO-d6 and $CDCl_3$. Mass spectrometric analyses were performed in the positive ion mode using a quadrupole mass spectrometer (micromass, Platform II).

***Electrospray ionization mass spectrometry (ESI-mass)*** spectrum were performed with SYNAPT G2-S (Waters Corporation, Manchester, UK) equipped with an ESI source was employed.

***High-resolution electrospray ionization mass spectrometry (HR-ESI-MS)*** were acquired in positive or negative ion mode. Conditions: capillary voltage 3000 V; cone voltage 20V, dry gas temperature 140 °C, desolvatation temperature = 450°C, dry gas flow, 1000 L.$h^{-1}$ and nitrogen as nebulizer gas, Pressure = 6.5 bars. 1ng/μl Leucine Enkephalin was used as standard for internal calibration.

***Thermogravimetric analysis (TGA)*** was measured by TA Instruments SDT Q600 by heating the sample to 700 °C under nitrogen (60 mL min-1) at a heating rate of 20 °C $min^{-1}$.

***Differential scanning calorimetry (DSC)*** was measured by TA Instruments Q20 by heating the sample to 700 °C under nitrogen (60 mL min-1) at a heating rate of 20 °C $min^{-1}$.

***The dynamic light scattering (DLS)*** experiments were performed using a Malvern Zetasizer Nano-S ZEN1600 with a 173° backscatter measurement angle and a quartz cuvette with a square aperture. The average size was calculated by using the hydrodynamic diameter values ($D_h$) obtained from at least three different measurements.

***FT-IR analysis*** was made on a Thermo Nicolet Nexus FTIR spectrometer with diamond ATR attachment.

Samples was subjected to 32 scans in the range of 4000 $cm^{-1}$ and 600 $cm^{-1}$.

***Synthesis of the compounds***

All of the compounds **(Δ, Δ.HCl, ΔC4, ΔC6, ΔC8 and ΔC12)** have been synthesized following the general scheme shown above:

**Synthesis of trianglamine (Δ):** A mixture of (±)-trans-1,2-diaminocyclohexane (2.28 g, 20 mmol), terephthalaldehyde (2.68g, 20 mmol) was added in methanol solution (200 mL) with triethylamine (7.0 mL, 50 mmol) and stirred overnight. The mixture was cooled in an ice bath and sodium borohydride (2.28 g, 60 mmol) was added over one hour to reduce trianglimine in trianglamine. After a stirring of three hours at room temperature, the solvents were removed under vacuum and the residue was dissolved in dichloromethane (100 ml) and washed with aqueous sodium carbonate (5%) and water. The organic extract was dried with magnesium sulfate and then evaporated and dried under vacuum at 50°C during five hours. Trianglamine was obtained in a yield of 4 g (90%). In order to obtain pure trianglamine, the macrocycle was solubilized with 4 mL of absolute EtOH. A solution of 10 mL of absolute EtOH with 2 mL of concentrated HCl (37%) was added dropwise to the absolute trianglamine mixture. The formation of white precipitate was observed during the addition. The solid was filtered and dried under vacuum. The white solid was dissolved in water and make it basic with (20 ml, 2 M NaOH) solution, which form a white precipitate during the addition. The pure precipitate trianglamine (Δ) was filtered and dried under vacuum desiccator for one day at 50 °C.

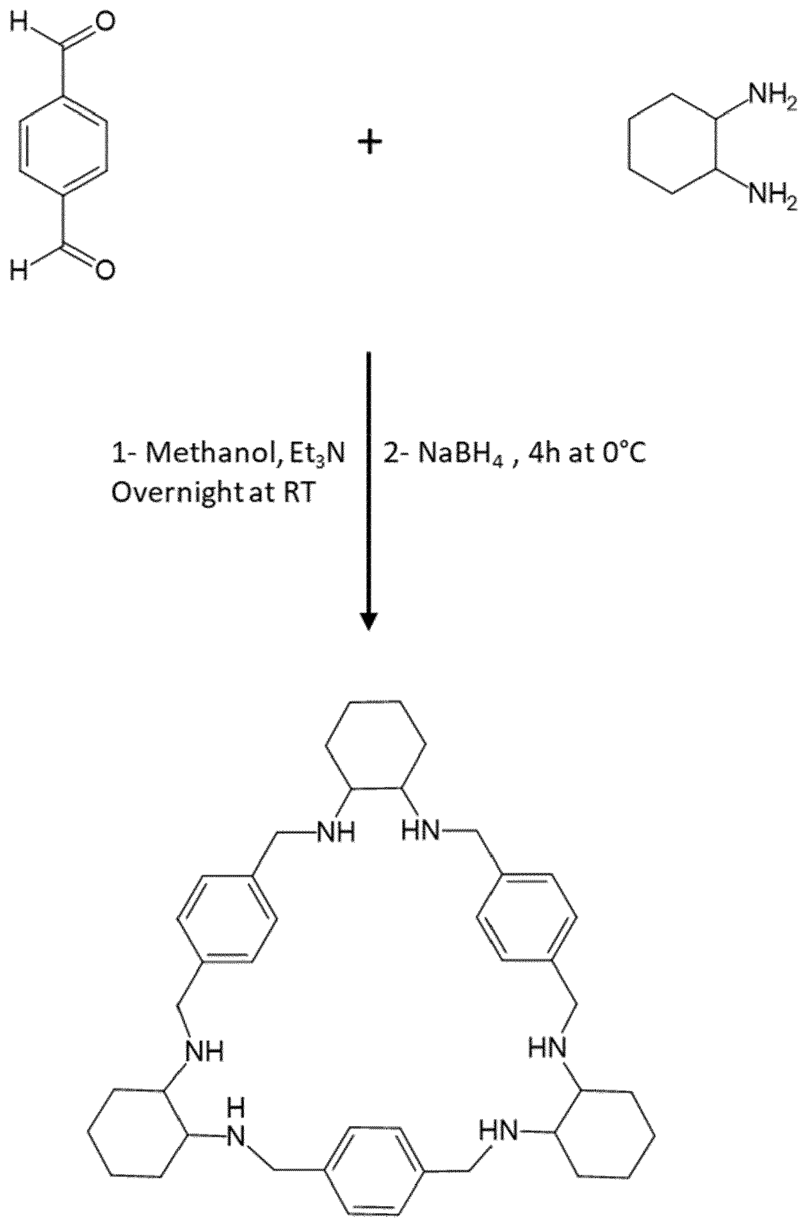


***Scheme 1**. Synthesis of the trianglamine (Δ).*

***Chemicals characterizations***

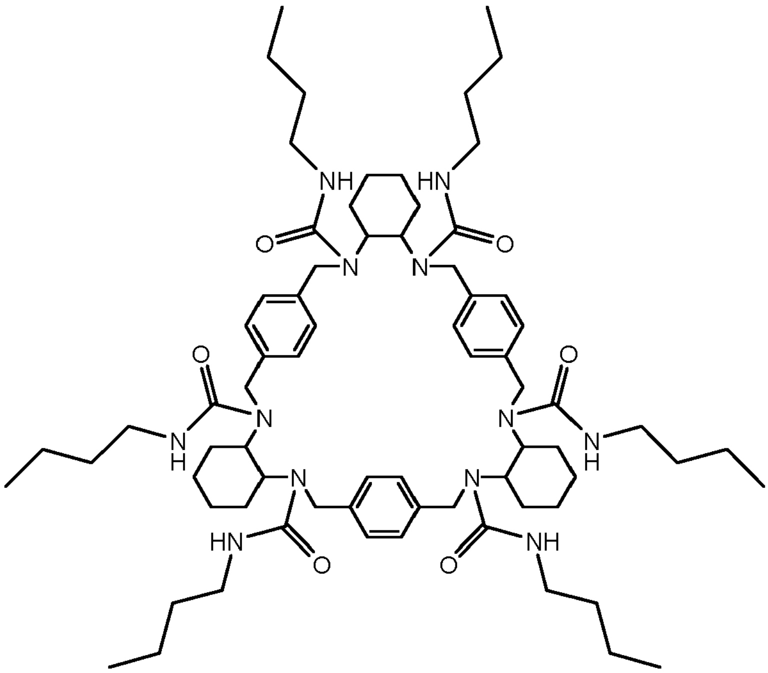

ΔC4

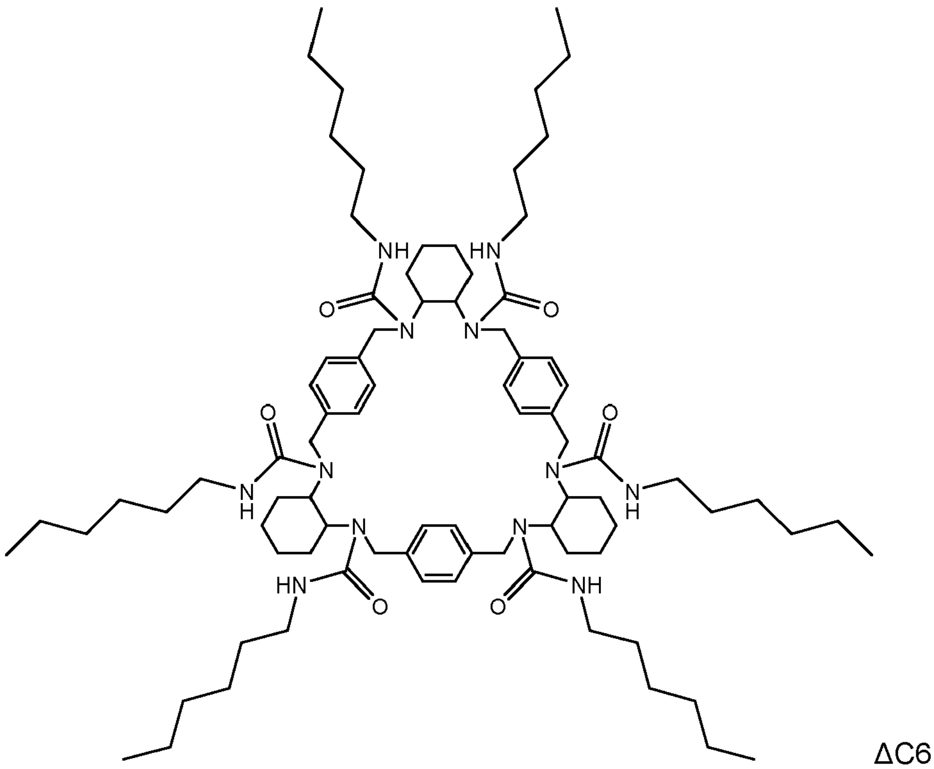

ΔC6

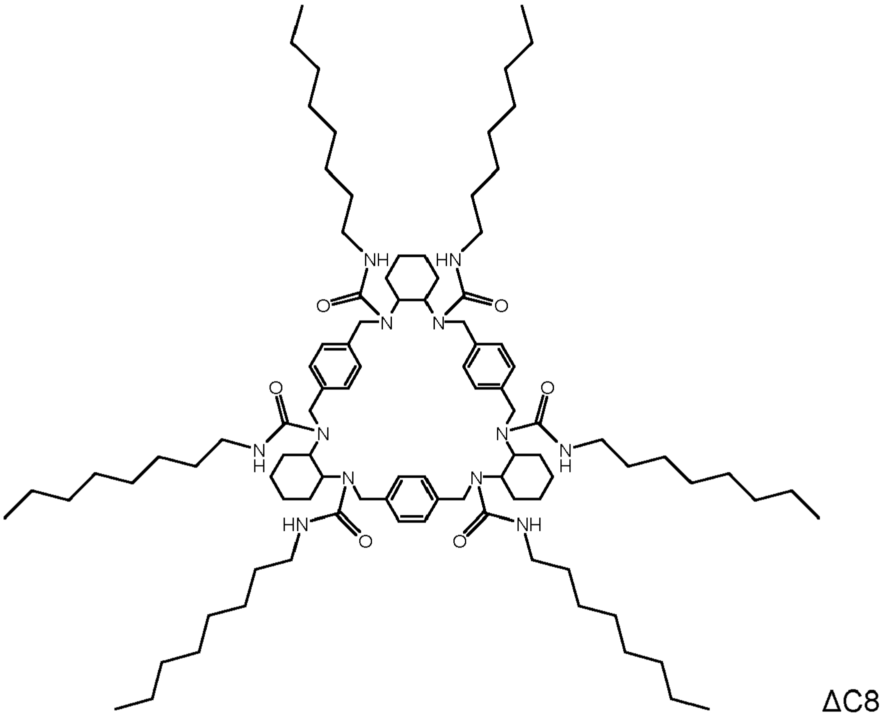

ΔC8

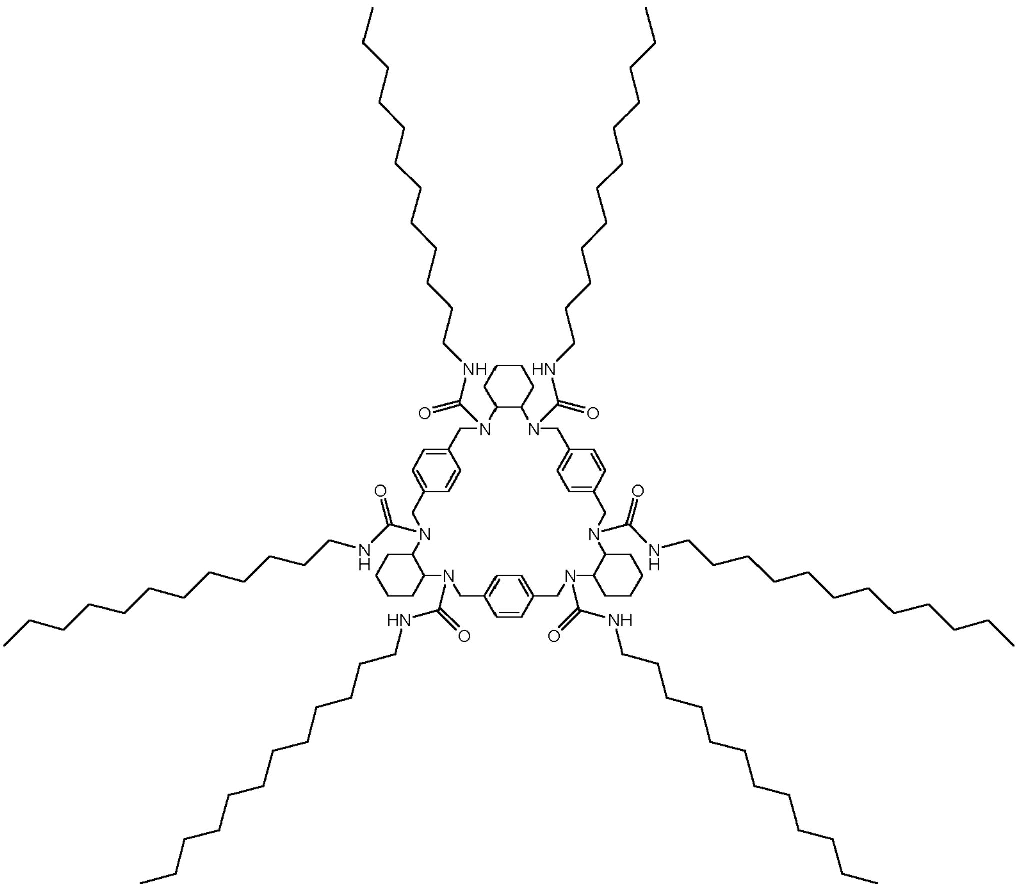

ΔC12

***Scheme 2.*** *Compounds according to the invention.*

**$^1H$, $^{13}C$ NMR, FTIR and MS Cal:**

**Trianglamine, Δ: $^1H$ NMR ($CDCl_3$, 400 MHz)** δ (ppm) = 7.3 (d, 12H), 3.9 (d, 6H), 3.6 (d, 6H), 2.30 (m, 6H), 2.25 (m,

6H), 1.75 (m, 6H), 1.1-1.3 (m, 12H); **$^{13}$C NMR (CDC13, 400 MHz)** δ (ppm) = 25, 32, 51, 61, 128, 139; **FTIR** (cm$^{-1}$) = 3290 (NH, stretch), 2922 ($C_{aromatic}$-H, stretch), 2850 ($C_{alkane}$-H, stretch), 1201 (C-N); **MS Cal.** = 648.5, ESI-MS found **$[M+H^+]^+$** = 649.5.

**Butyl-urea trianglamine, ΔC4: $^1$H NMR (DMSO-*d6*, 500 MHz at 100°C)** δ (ppm) = 6.90- 7.15 (m, 12H), 6.2 (s, 6H), 3.93 (m, 12H), 3.01-3.12 (m, 12H), 1.39-1.45 (m, 12H), 1.28-1.30 (m, 12H), 1.11-1.17 (m, 6H), 0.87-0.9 (m, 18H); **$^{13}$C NMR (CDCl3, 400 MHz)** δ (ppm) = 12, 20, 26, 32, 41, 54, 57, 126, 129, 160; **FTIR** (cm$^{-1}$) = 3307 (NH, stretch), 2927 ($C_{aromatic}$-H, stretch), 2860 ($C_{alkane}$-H, stretch), 1620 (C=O, stretch, NH-CO-NH), 1535 (N-H, bending), 1246 (C-N); **MS Cal.** = 1242.9, ESI-MS found **$[M+H^+]^+$** = 1243.9.

**Hexyl-urea trianglamine, ΔC6: $^1$H NMR (DMSO-*d6*, 500 MHz at 100 °C)** δ (ppm) = 6.90- 7.15 (m, 12H), 6.2 (s, 6H), 3.93 (m, 12H), 3.01-3.12 (m, 12H), 1.39-1.45 (m, 12H), 1.28-1.30 (m, 36H), 1.11-1.17 (m, 6H), 0.87-0.9 (m, 18H); **$^{13}$C NMR (CDC13, 400 MHz)** δ (ppm) = 14, 20, 25, 26, 29, 32, 41, 45, 55, 57, 126, 129, 160; **FTIR** (cm$^{-1}$) = 3311 (NH, stretch), 2925 ($C_{aromatic}$-H, stretch), 2856 ($C_{alkane}$-H, stretch), 1622 (C=O, stretch, NH-CO-NH), 1537 (N-H, bending), 1246 (C-N); **MS Cal.** = 1411.09, ESI-MS found **$[M+H^+]^+$** = 1412.09.

**Octyl-urea trianglamine, ΔC8: $^1$H NMR (DMSO-*d6*, 500 MHz at 100 °C)** δ (ppm) = 6.90- 7.15 (m, 12H), 6.2 (s, 6H), 3.93 (m, 12H), 3.01-3.12 (m, 12H), 1.39-1.45 (m, 12H), 1.28-1.30 (m, 60H), 1.11-1.17 (m, 6H), 0.87-0.9 (m, 18H); **$^{13}$C NMR (CDC13, 400 MHz)** δ (ppm) = 14, 22, 25, 26, 27, 29, 30, 31, 41, 45, 55, 57, 126, 129, 160; **FTIR** (cm$^{-1}$) = 3321 (NH, stretch), 2924 ($C_{aromatic}$-H, stretch), 2854 ($C_{alkane}$-H, stretch), 1624 (C=O, stretch, NH-CO-NH), 1541 (N-H, bending), 1246 (C-N); **MS Cal.** = 1580.3, ESI-MS found **$[M+H^+]^+$** = 1581.3.

**Dodecyl-urea trianglamine, ΔC12: $^1$H NMR (DMSO-*d6*, 500 MHz at 100 °C)** δ (ppm) = 6.90- 7.15 (m, 12H), 6.2 (s, 6H), 3.93 (m, 12H), 3.01-3.12 (m, 12H), 1.39-1.45 (m, 12H), 1.28-1.30 (m, 120H), 1.11-1.17 (m, 6H), 0.87-0.9 (m, 18H); **$^{13}$C NMR (CDC13, 400 MHz)** δ (ppm) = 14, 22, 25, 26, 27, 28, 29, 31, 41, 45, 54, 57, 126, 129, 160; **FTIR** (cm$^{-1}$) = 3327 (NH, stretch), 2922 ($C_{aromatic}$-H, stretch), 2852 ($C_{alkane}$-H, stretch), 1626 (C=O, stretch, NH-CO-NH), 1541 (N-H, bending), 1248 (C-N); **MS Cal.** = 1917, ESI-MS found **$[M+H^+]^+$** = 1917.7

**Example 2: Preparation of crosslinked material**

***Materials* & *methods***

All reagents were purchased from commercial suppliers and used without further purification. (±)-trans-1,2-diaminocyclohexane, terephthalaldehyde, triethylamine, sodium borohydride, Polypropylene glycol) toluene 2,4-diisocyanate ( $M_n$~2300 g/mol) and anhydrous chloroform were purchased from Sigma Aldrich.

Synthesis of trianglamine (Δ):

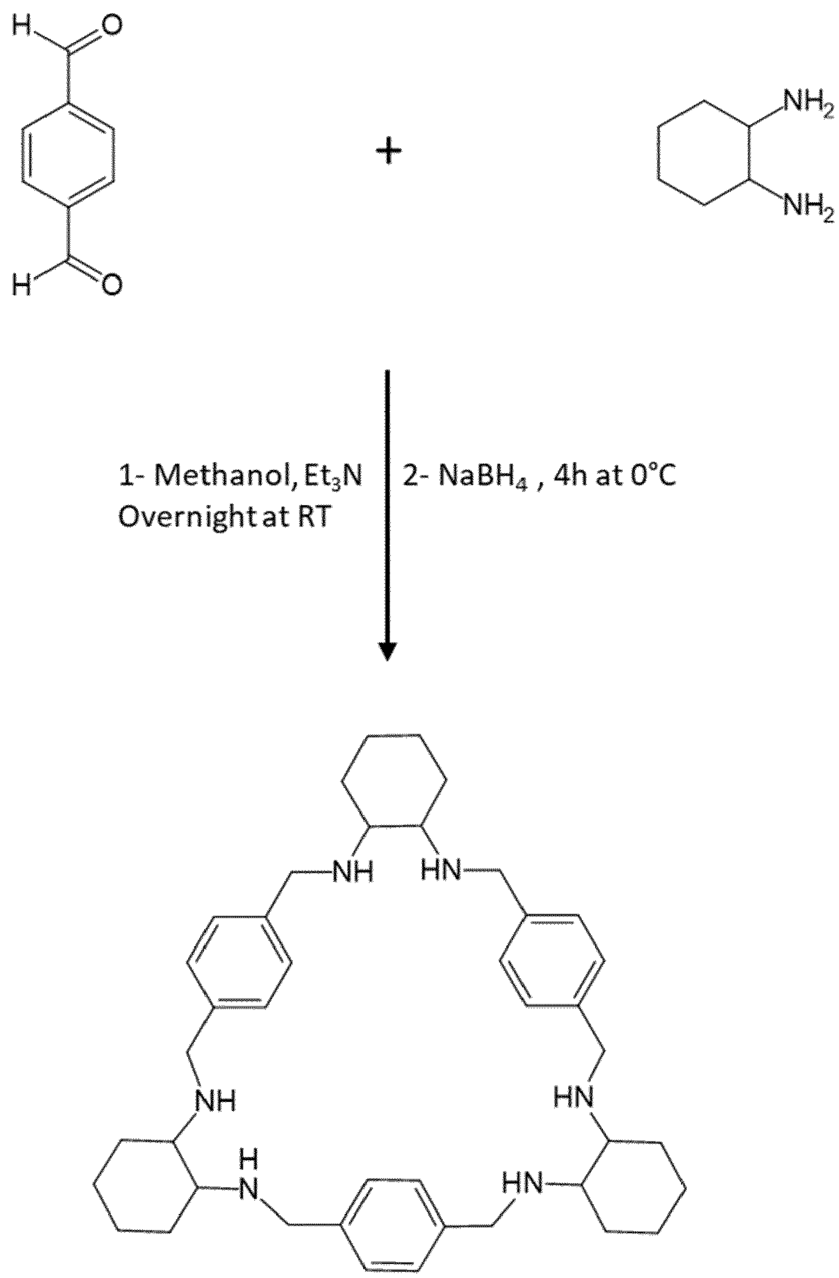


***Scheme 3.*** *Synthesis of the trianglamine (Δ).*

A mixture of (±)-trans-1,2-diaminocyclohexane (2.28 g, 20 mmol), terephthalaldehyde (2.68g, 20 mmol) was added in methanol solution (200 mL) with triethylamine (7.0 mL, 50 mmol) and stirred overnight. The mixture was cooled in an ice bath and sodium borohydride (2.28 g, 60 mmol) was added over one hour to reduce trianglimine in trianglamine. After a stirring of three hours at room temperature, the solvents were removed under vacuum and the residue was dissolved in dichloromethane (100 ml) and washed with aqueous sodium carbonate (5%) and water. The organic extract was dried with magnesium sulfate and then evaporated and dried under vacuum at 50°C during five hours. Trianglamine was obtained in a yield of 4 g (90%). In order to obtain pure trianglamine, the macrocycle was solubilized with 4 mL of absolute EtOH. A solution of 10 mL of absolute EtOH with 2 mL of concentrated HCl (37%) was added dropwise to the absolute trianglamine mixture. The formation of a white precipitate was observed during the addition. The solid was filtered and dried under vacuum. The white solid was dissolved in water and make it basic with (20 ml, 2 M NaOH) solution, which form a white precipitate during the addition. The pure precipitate trianglamine (Δ) was filtered and dried under vacuum desiccator for one day at 50 °C.

**NMR $^1$H ($CDCl_3$, 400 MHz)** δ (ppm) = 7.3 (d, 12H), 3.9 (d, 6H), 3.6 (d, 6H), 2.30 (m, 6H), 2.25 (m, 6H), 1.75 (m,

6H), 1.1-1.3 (m, 12H).
**NMR $^{13}C$ $(CDC)_3$, 400 MHz)** $\delta$ (ppm) = 25, 32, 51, 61, 128, 139.
**MS Cal.** = 648.5, ESI-MS found **$[M+H^+]^+$** = 649.5.

***Preparation of gels***

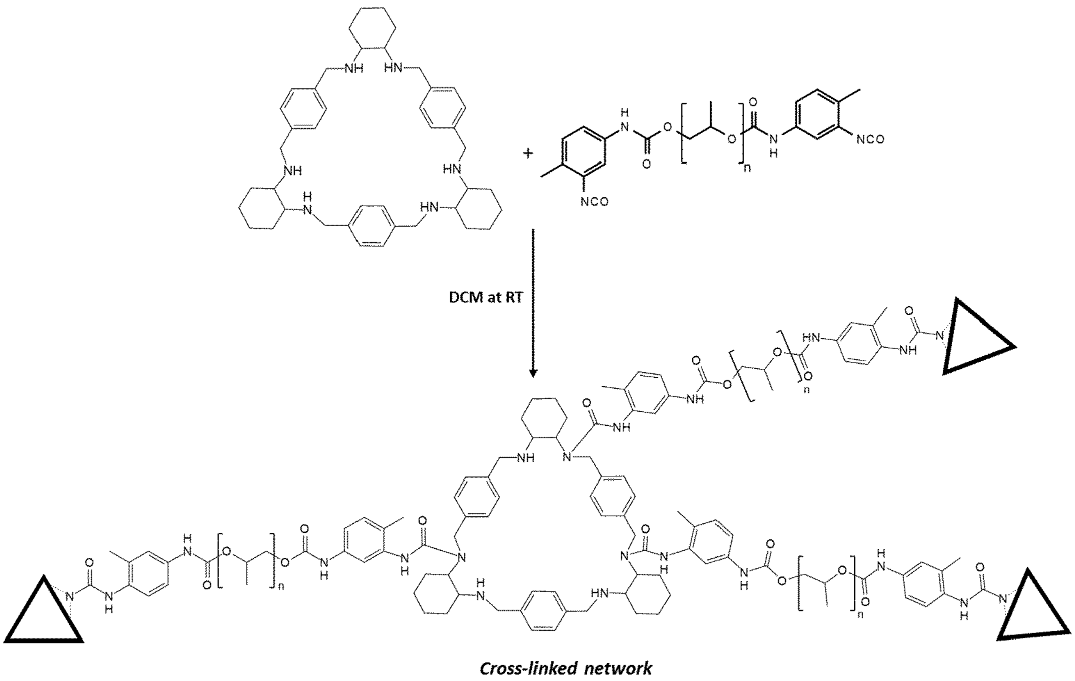


***Scheme 4:*** *Fabrication of gels by cross-linking reaction between isocyanate of PPG and Amine function of Δ*

Two gels with different cross-linked rate were prepared as follow. In a first glass vial of 5 mL we dissolved 100 mg of trianglamine(Δ) in 0.5mL of dichloromethane. And in a second vial, we dissolved 3eq or 7 eq of poly (propylene glycol) 2,4-diisocyanate ($M_n$~2300 g/mol) in 1 mL of dichloromethane. Both vials were placed in ice for 15 min, in order to slow down the reactivity. The trianglamine solution was quickly added to PPG solution, agitated by hand, and closed. In less than 1 minute the gel is formed, this last was left inside for 24 hours to be sure that all isocyanate has been reacted. In the end, the gels were washed in dichloromethane to remove the unreacted reagents (PPG and trianglamine), the solvent was exchanged every 12 hours for 3 days.

**Characterization of hydrogel materials**

**Swelling degree (SD) and equilibrium swelling degree (ESD) measurement**

The swelling behavior of the gels (Δ@PPG-6, Δ@PPG-3) was determined by placing a weighed dry polymer network (Wx) into de-ionized water, dimethyl sulfoxide (DMF) or dichloromethane (DCM) at room temperature (25 °C). The swollen gels were removed from the solvents bath at specific time intervals, wiped superficially with filter paper, weighed (Wy), and sunk back into the corresponding solvents. The measurements were carried out until the weight of the swollen gel reached a constant value, corresponding to the equilibrium swelling degree (ESD). The experiments were done three times, and the average value measurements were reported. The swelling degree at various time intervals, expressed as the amount of solvent absorbed by the dry gel, was calculated using Equation (1). The ESD was determined as the value corresponding to the curve plateau of the swelling degree (SD).

$$SD (\%) = (\frac{Wy-Wx}{Wy})*100 \qquad (1)$$

where $W_x$ is the weight of the gel (before swelling), and $W_y$ the weight of the swollen hydrogel at different times t (hours).

**Table 5:** The equilibrium swelling degree (ESD) of both gels ((∆@PPG3, ∆@PPG7) in three (03) different solvents.

| | Equilibrium swelling degree (ESD%) | | |
|---|---|---|---|
| Gels/solvants | Water | Dimethylformamide | Dichloromethane |
| Gel ∆@PPG3 | 48% | 298% | 569% |
| Gel ∆@PPG6 | 29 % | 211% | 497% |

**Determination of gel fraction**

In order to determine the cross-linking efficiency, the gel fraction of the two gels was evaluated as follows. The gel was first dried to constant weight ($W_0$) and purified by immersing it in dichloromethane for 24 hours. After this step, the gel was dried again to record the final weight ($W_{ext}$). The gel fraction (GF) was calculated according to Equation 2:

$$GF\% = (\frac{Wext}{W0})\times 100 \qquad (2)$$

**Table 6:** Gel Fraction.

| | ∆@PPG3 | ∆@PPG6 |
|---|---|---|
| Gel fractions (%) | 86% | 97% |

**FTIR characterization of gels**

FTIR Spectroscopy verified the formation of the gel by stacking of the spectrums of the trianglamine, PPG, and gels. We noticed the appearance of a urea function of N-C and C=O bonds at 1550 $cm^{-1}$ and 1700 $cm^{-1}$ respectively. Moreover, we noticed the disappearance of the bond characteristic of the isocyanate function of the PPG around 2250 and 2275$cm^{-1}$. The other peaks of the gels correspond to the characteristic parts of the PPG and the trianglamine with specific shifts at 1100$cm^{-1}$ (C-O), 1200$cm^{-1}$ (C-N), 1400$cm^{-1}$ (C-H), 2900$cm^{-1}$ (C-H) and 3200$cm^{-1}$ (N-H).

**Iodine uptake experiments in aqueous solution**

In order to monitor the $I_2$ capture speed of gels in an aqueous solution, a time-dependent UV-Vis measurement was carried out on a UV-Vis spectrophotometer. Gels (20 mg) were added to a saturated $I_2$ aqueous solution (V= 50 mL, C= 30 mmol.$L^{-1}$). The UV-Vis spectrum of the solution was recorded every 10 minutes in the first 30 mins, and at 1 h, 1h30, 2h30, and 4h30, respectively. The $I_2$ concentration at different intervals was calculated using a standard curve. See figures 1 and 2.

**Cycles of adsorption and desorption efficiency**

In order to monitor the iodine-releasing from iodine-loaded gels, time-dependent UV-visble measurements were carried out in DMF. In a typical experiment, iodine-loaded gels were immersed in DMSO in a reaction vial with stirring. The UV-Vis adsorptions were recorded by periodically withdrawing a from the mother solution and then transferred back. The experiment of uptake and realize of iodine was repeated several times (5 times) in order to monitor the efficiency of adsorption/desorption (figure 3).

**Maximum iodine uptake in water and seawater**

Pieces of gels were placed in 20mL of 0.2M iodine solution to determine the maximum uptake of iodine that

the gels can uptake. After 7 days, the solution was diluted and analyzed by UV-visible spectroscopy. The final concentration and the weight of iodine were determined using a calibration curve. See figure 4.

**Iodine uptake experiments in the vapors phase**

The iodine vapor adsorption capacities of gels were calculated by mass measurements. Gels and trianglamine powder were introduced into a vessel, which contained an amount of solid $I_2$. Then the vessel was then sealed and heated using an oven at 75°C (figure x) for 0.5-96 h to allow the adsorption of iodine by samples. The iodine-loaded samples were cooled to room temperature and collected for further analysis. The iodine capture of gels was evaluated by the following equation: $(W_2 - W_1)/W_1$. Where $W_1$ and $W_2$ represent the mass of gels before and after iodine capture, respectively. See figure 5.

**Example 3: 3D Printed Hydrogels Based on Trianglamine and Pluronic for Highly Efficient Iodine Capture in Aqueous and Vapor Phase**

**Synthesis of Trianglamine di-methacrylate (∆@di-MMA). The** trianglamine 3.72 g (0.0058 mol) was dissolved in 60 mL of dichloromethane and added in round bottom flask. Then, a solution of 2 equivalences of the 2-Isocyanatoethyl methacrylate (1.81 g, 0.0116 mol) in 10 mL of dichloromethane was added drop by drop to the round bottom flask. The reaction mixture was stirred overnight at room temperature. Then, the solvent (dichloromethane) was removed under vacuum and dried under reduced pressure. to lead to the desired product with a yield of 98%. **Trianglamine**, **∆@MMA: $^1$H NMR ($CDCl_3$, 400 MHz)** δ (ppm) = 7.3 (d, 12H), 6.01 (d, 1H), 5.51 (d, 1H), 4.16 (t, 2H) ( 3.9 (d, 6H), 3.6 (d, 6H), 3.4 (t, 2H), 2.30 (m, 6H), 2.25 (m, 6H), 1.87 (t, 3H), 1.75 (m, 6H), 1.1-1.3 (m, 12H); **$^{13}$C NMR ($CDCl_3$, 400 MHz)** δ (ppm) =18.4, 24.64, 25.09, 25.77, 32, 40, 51, 61, 125, 127, 128, 136, 139, 167 ;**MS Cal.** = 957.6, ESI-MS found **$[M+H^+]^+$** = 959.6

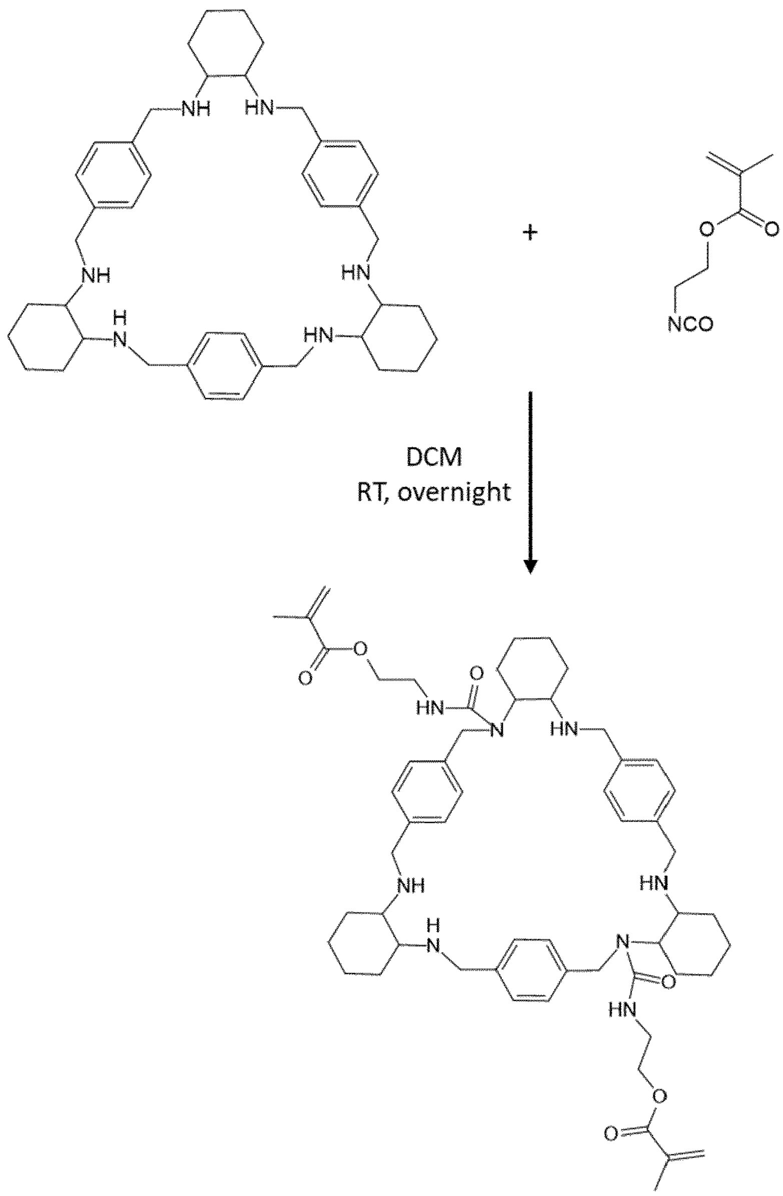


**Scheme 5.** Synthesis of the trianglamine di-methacrylate (Δ@di-MMA)

**Fabrication of 3D printed hydrogel by SLA (stereolithography)**

The resin formulation was prepared for the SLA process by mixing trianglamine di-methacrylate (Δ@*di-MMA*), pluronic-dimethacrylate and darocur 1173 with a non-reactive solvent (DMSO) at 50 wt%. The solution was stirred overnight at 25 °C and yielded a homogeneous reactive solution providing an optimal pattern resolution. 3D cylinder was designed from Rhinoceros 3D using the STL format. The 3D structure was built by SLA from the resin mentioned above by using a 385 nm digital light processing (DLP) apparatus (Asiga Max X43, Australia). The 3D objects were constructed layer-by-layer by photo-crosslinking with a thickness of 200 μm. Each layer was illuminated over an intensity of 40 mW/cm2 for a specific duration of 60 s. After building, the object was washed with DMSO, then water, and dried at room temperature for 48h.

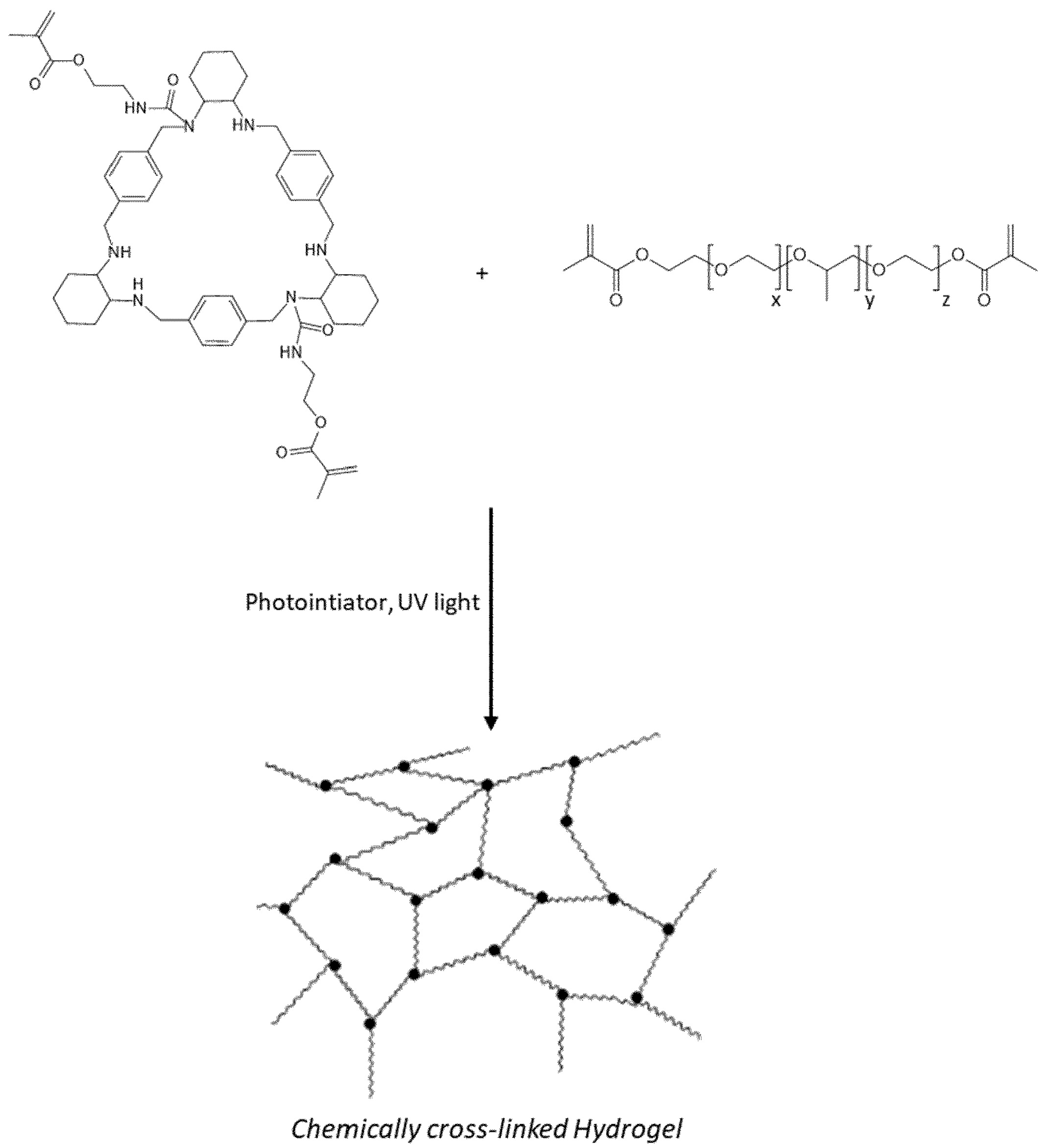


**Scheme 6.** 3D Fabrication of the hydrogels based on trianglamine and pluronic

**Example 4: Polymeric membranes based on Trianglamine and poly( methyl methacrylate for selective gas separation)**

**Synthesis of Trianglamine di-methacrylate (△@di-MMA).** The trianglamine 3.72 g (0.0058 mol) was dissolved in 60 mL of dichloromethane and added in round bottom flask. Then, a solution of 2 equivalences of the 2-Isocyanatoethyl methacrylate (1.81 g, 0.0116 mol) in 10 mL of dichloromethane was added drop by drop to the round bottom flask. The reaction mixture was stirred overnight at room temperature. Then, the solvent (dichloromethane) was removed under vacuum and dried under reduced pressure. to lead to the desired product with a yield of 98%. **Trianglamine, △@MMA: $^1$H NMR ($CDCl_3$, 400 MHz)** $\delta$ (ppm) = 7.3 (d, 12H), 6.01 (d, 1H), 5.51 (d, 1H), 4.16 (t, 2H) ( 3.9 (d, 6H), 3.6 (d, 6H), 3.4 (t, 2H), 2.30 (m, 6H), 2.25 (m, 6H), 1.87 (t, 3H), 1.75 (m, 6H), 1.1-1.3 (m, 12H); **$^{13}$C NMR ($CDCl_3$, 400 MHz)** $\delta$ (ppm) =18.4, 24.64, 25.09, 25.77, 32, 40, 51, 61, 125, 127, 128, 136, 139, 167 ;**MS Cal.** = 957.6, ESI-MS found **$[M+H^+]^+$** = 959.6

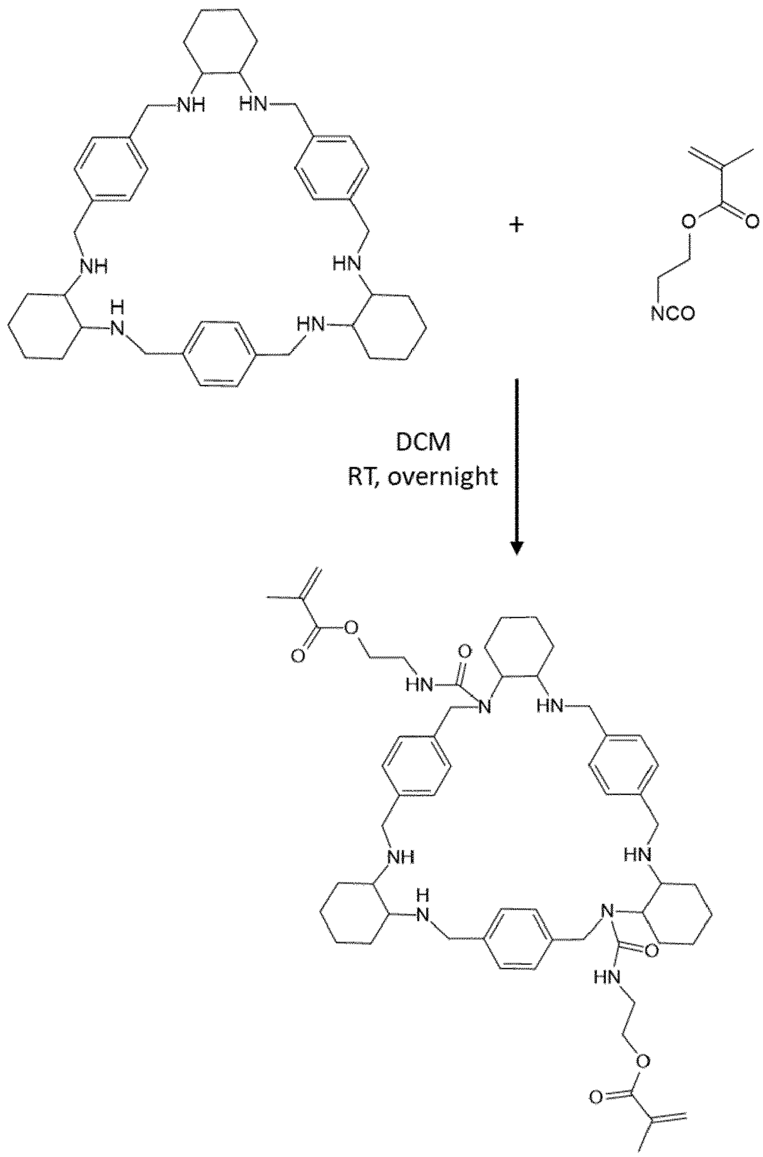


**Scheme 7**. Synthesis of the trianglamine di-methacrylate (Δ@di-MMA)

**Example 5: Membrane** Fabrication

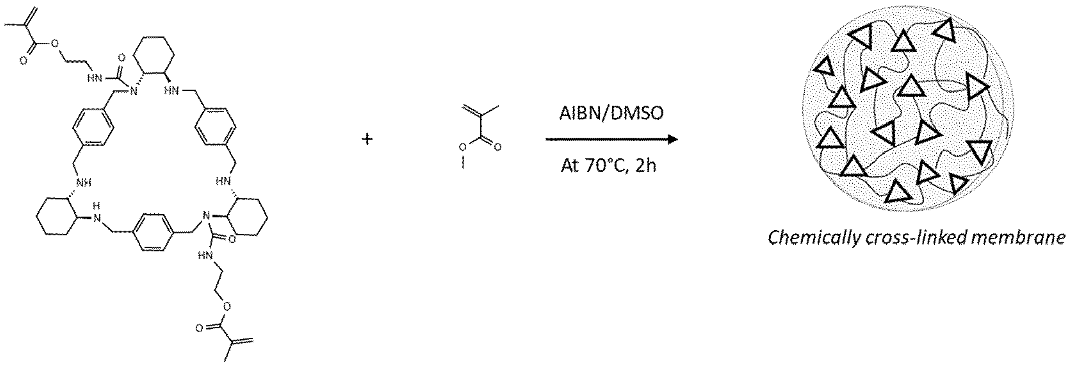

0.57 g (6×$10^{-3}$ mmol, 1eq) of (Δ@di-MMA), 6 g (0.6.$10^{-3}$×mmol, 100eq) of methyl methacrylate, and 99.4 mg (0.06×$10^{-5}$ mmol, 0.1 eq) of AIBN were dissolved in 6.6 mL of DMSO. 15 min of stirring was required to obtain the complete dissolution of the reactants. The solution was poured into a Teflon round flat inner. The mold was put in an oven at 70°C for 1h, then the film/membrane was washed in DMSO 3 times in order to remove the residual solvent and unreacted methacrylate. Finally, the membrane/film was dried in a vacuum oven at 50°C for 48 h.

Example 6: Gels based on trianglamine@Poly (propylene glycol) for the adsorption of PFAs and PFOA

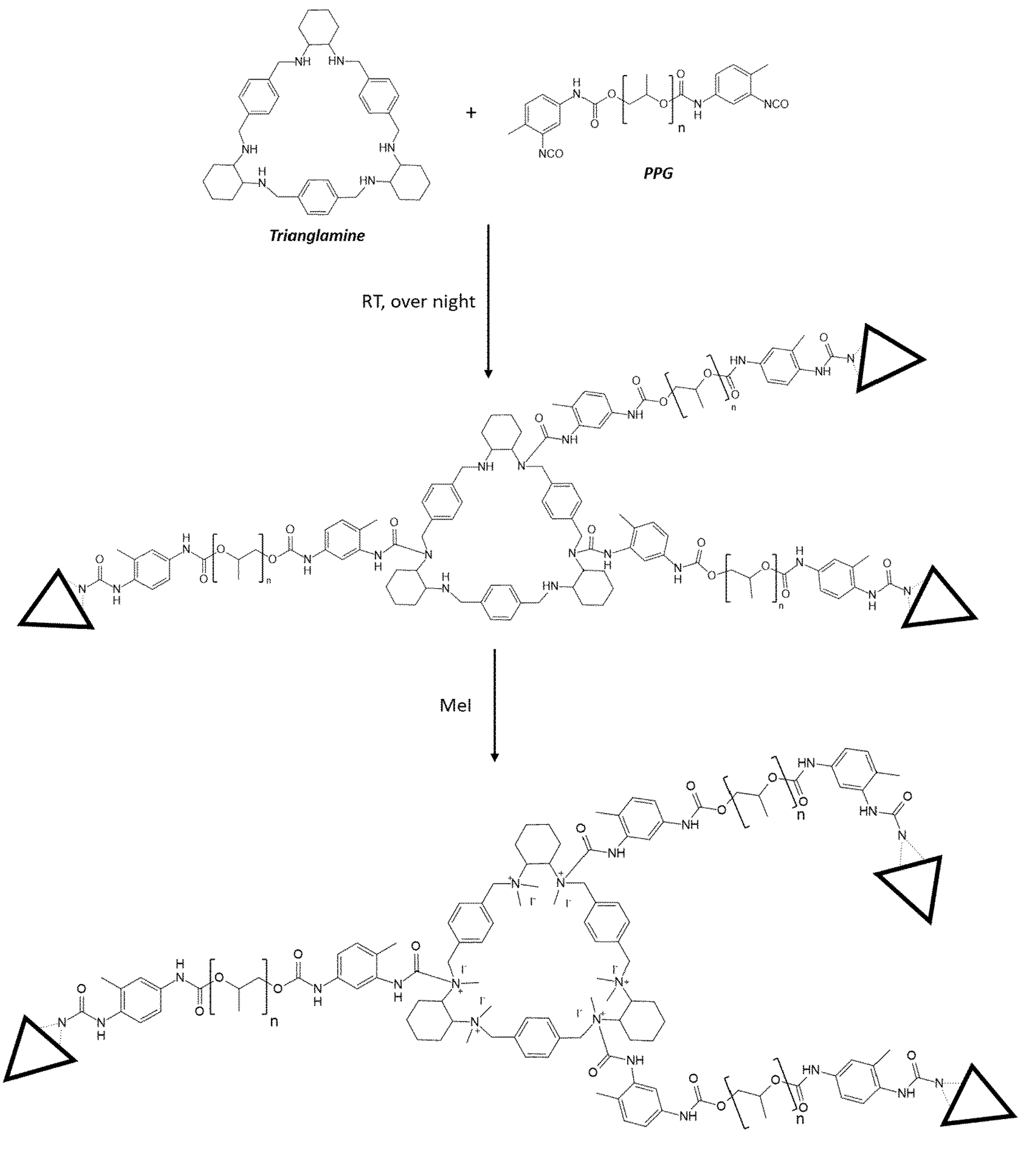


*Quaternized Gels*

***Fabrication of the gel***

***Step1*** In a first glass vial of 5 mL we dissolved 100 mg of trianglamine(Δ) in 0.5mL of dichloromethane. And in a second vial, we dissolved 3eq of poly (propylene glycol) 2,4-diisocyanate ($M_n$~2300 g/mol) in 1 mL of dichloromethane. Both vials were placed in ice for 15 min, in order to slow down the reactivity. The trianglamine solution was quickly added to PPG solution, agitated by hand, and closed. In less than 1 minute the gel is formed, this last was left inside for 24

hours to be sure that all isocyanate has been reacted. In the end, the gels were washed in dichloromethane to remove the unreacted reagents (PPG and trianglamine), and the solvent was exchanged every 12 hours for 3 days and dried at room temperature for 48h.

***Step2*** The resulting gel from the step1 was immersed in 20mL of DCM containing 2 mL of iodomethane (MeI). the gel was left inside for 24 hours to be sure that all amine functions get quaternized. In the end, the gels were washed in dichloromethane to remove the unreacted iodomethane), and the solvent was exchanged every 12 hours for 3 days and dried at room temperature during 48h.

**PFOA adsorption experiments**

Adsorption experiments were conducted at room temperature. 20 mg of the gels were placed in a beaker containing 50 mL PFOA (aq.) solution of 100 ppm. The solution was stirred for 6 days, and samples were taken at different time intervals. The adsorption kinetics were analyzed by pseudo-first order model and pseudo-second order model by fitting the experimental data into their linear form consecutively (Eq. 1) and (Eq. 2):

$$\mathbf{ln(q_e - q_t) = ln(q_e) - K_1 t} \ldots\text{Eq.1}$$

$$\frac{t}{q_t} = \frac{1}{K_2\, q_e^2} + \frac{t}{q_e} \ldots\text{Eq.2}$$

Where $q_e$ is defined as the equilibrium adsorption capacity, $K_1$ (min-1) is the constant of the pseudo-first order model, and $K_2$(g $mg^{-1}$ $min^{-1}$) is the constant of the pseudo-second order model.

A concentration ranging from 1 to 100 ppm of PFOA was used to investigate adsorption isotherms based on Langmuir and Freundlich model. Langmuir model considers monolayer adsorption of the adsorbate and homogeneous distribution of the adsorption site. The following equation presents the linear form:

$$\frac{C_e}{q_e} = \frac{C_e}{q_m} + \frac{1}{q_m K_L} \ldots\ldots\text{Eq.3}$$

Where C_e is the concentration at the equilibrium, $q_e$ is the quantity adsorbed at the equilibrium, $q_m$ is the maximum quantity adsorbed, and $K_L$ is the Langmuir constant.

Freundlich model considers the binding sites of the adsorbent heterogeneous, and the adsorption is multilayer. The following equation presents the linear form:

$$\ln(q_e) = \ln K_f + \frac{1}{n}\ln C_e \ldots\ldots\text{Eq.4}$$

Where $K_f$ is a Freundlich constant, and 1/n is the heterogeneity factor. See figure 6.

**List of references**

[1] B. Valizadeh, T. N. Nguyen and K. C. Stylianou, Polyhedron, 2018, 145, 1-15.
[2] WO2022/058961.

**Claims**

**1.** A trianglamine based compound of formula I:

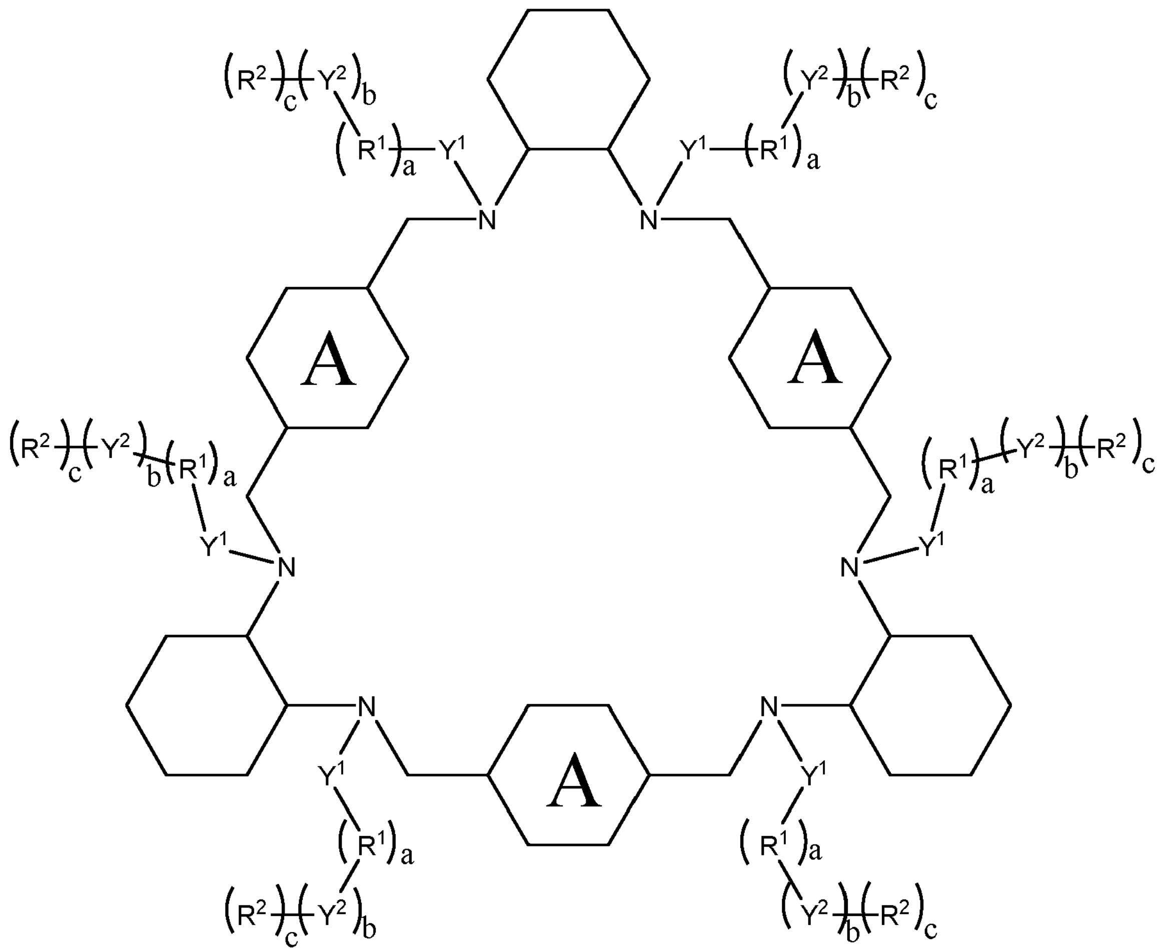

Formula I

wherein,

- each A independently represents a substituted or unsubstituted 4 to 18-membered aryl or heteroaryl linker,
- each $Y^1$ independently represents a bond or forms, together with the nitrogen of the trianglamine, a functional group chosen from urea (-N-C(O)-NH-), carbamate (-N-C(O)-O-) and amide (-N-C(O)-),
- each $R^1$ independently represents a mono or bifunctional radical chosen from H, a linear or cyclic, optionally branched, saturated or unsaturated, optionally substituted $C_1$ to $C_{30}$ hydrocarbon chain optionally comprising a heteroatom such as O or N, an optionally substituted $C_1$ to $C_{20}$ alkylaryl, an optionally substituted $C_1$ to $C_{20}$ alkylheteroaryl group, a substituted or unsubstituted C4 to C6-membered aryl or heteroaryl group or a polyether comprising from 2 to 100 monomers,
- at least one $-Y^1-(R^1)_a-(Y^2)_b-(R^2)_c$ radical is different from H,
- each a = 0 or 1,
- each b = 0 or 1,
- each c = 0 or 1,
- in a substituent $-Y^1-(R^1)_a-(Y^2)_b-(R^2)_c$ at least one from a, b and c is different from 0,
- each $Y^2$ independently represents a functional group chosen from hydroxyl (-OH), ester (-C(O)O- or -O-C(O)-), diester ($-O-C(O)-R^5-C(O)-O-$, in which $R^5$ is a $C_1$ to $C_8$ alkyl chain), carbamate (-NH-C(O)-O- or -O-C(O)-NH-), ether (-O-), amine (-NH- or -NR- in which R is a protecting group such as Boc), acrylate ($CH_2$=CHC(O)O-), methacrylate ($CH_2=C(CH_3)C(O)O-$), $-SiOR^4_3$ in which $R^4$ is a $C_1$ to $C_4$ alkyl chain, halo group, a maleinimido group, a silicagel and a resin,
- each $R^2$ independently represents a linker of formula II:

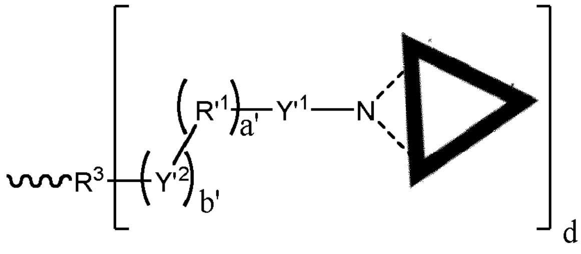

Formula II

wherein,

- $R^3$ represents a mono-, bi-, tri- or tetrafuntional radical comprising from 1 to 500 carbon atoms, optionally comprising hetero atoms such as O, N, Si or halo,
- the 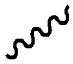 represents the point of attachment to $Y^2$ and

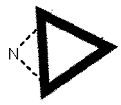

represents the point of attachment to another trianglamine based compound of formula I,
- $Y'^2$ is defined as $Y^2$, $R'^1$ is defined as $R^1$, $Y'^1$ is defined as $Y^1$, a' = 0 or 1, b' = 0 or 1 and d is an integer from 0 to 3.

**2.** The compound according to claim 1, $R^3$ is different from a cyclic alkyl or different from an aryl, when c = 1, d ≠ 0 and a = a' = b = b' = 0.

**3.** The compound according to claim 1 or 2, wherein A is chosen from a substituted or unsubstituted phenyl, a substituted or unsubstituted biphenyl, a substituted or unsubstituted triphenyl, a substituted or unsubstituted terphenyl, a substituted or unsubstituted diphenyl ether, a substituted or unsubstituted naphthalene, a substituted or unsubstituted phenanthrene, a substituted or unsubstituted anthracene and a substituted or unsubstituted pyridinyl, preferably a substituted or unsubstituted phenyl.

**4.** The compound according to any of preceding claims, wherein A is chosen from the following structures:

| | |
|---|---|
| 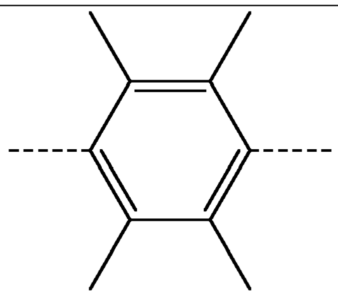 | 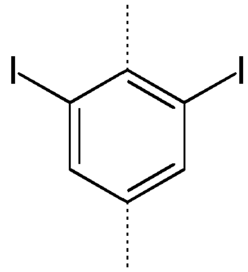 |
| 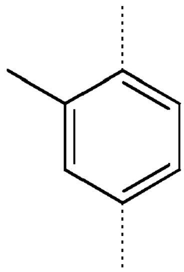 | 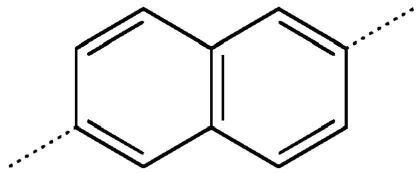 |

| | |
|---|---|
| 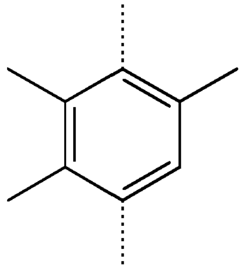 | 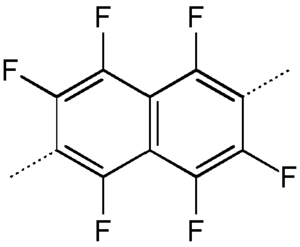 |
| 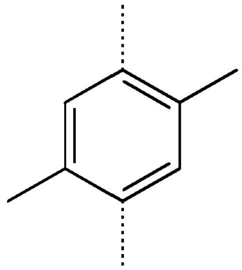 | 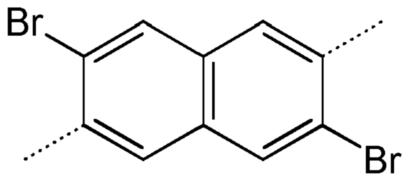 |
| 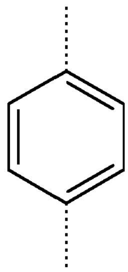 | 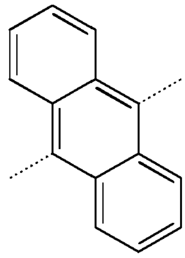 |
| 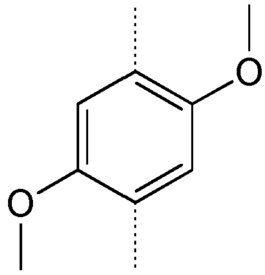 | 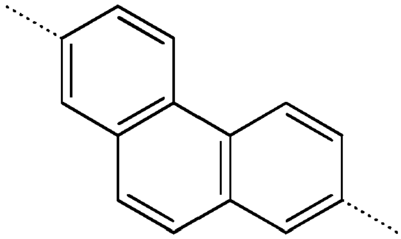 |
| 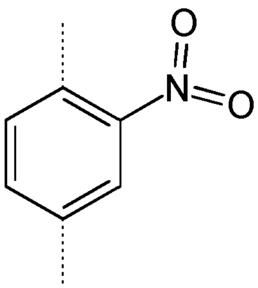 | 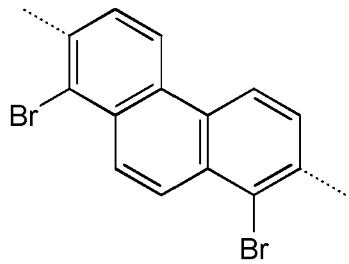 |

| | |
|---|---|
| 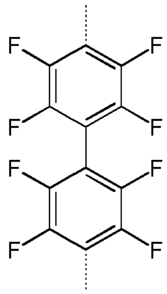 | 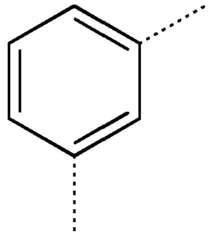 |
| 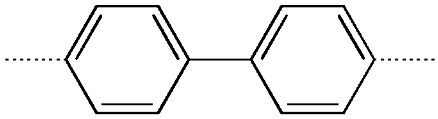 | 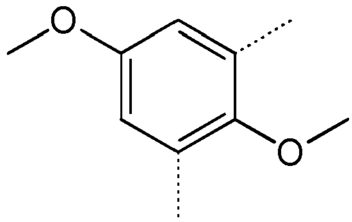 |
| 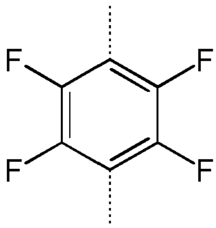 | 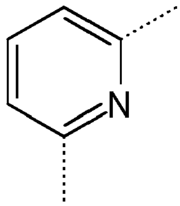 |
| 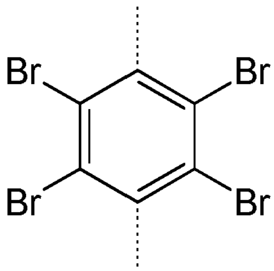 | 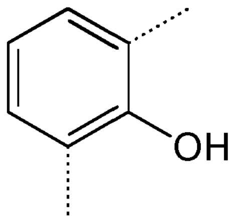 |
| 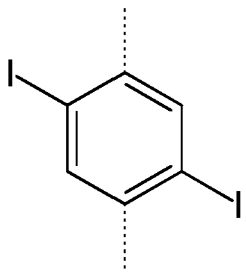 | 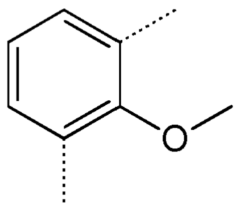 |

| | |
|---|---|
| 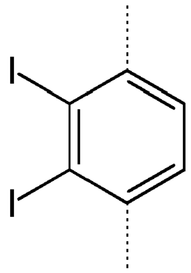 | 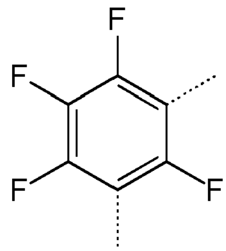 |
| | 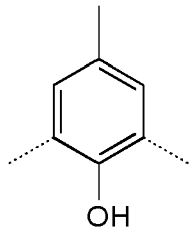 |

5. The compound according to any of preceding claims, wherein $R^1$ is H or a $C_2$ to $C_{22}$ linear alkyl chain, preferably H or a $C_4$ to $C_{12}$ linear alkyl chain, and more preferably chosen from H, butyl, hexyl, octyl and dodecyl.

6. The compound according to any of preceding claims, wherein $R^1$ is H, a substituted or unsubstituted $C_4$ to $C_6$-membered aryl or heteroaryl linker, or a polyether comprising from 2 to 100 monomers, preferably a methyl substituted phenyl.

7. The compound according to any of preceding claims, wherein $R^3$ is chosen from an optionally substituted linear or branched, saturated or unsaturated, $C_1$ to $C_{22}$ hydrocarbon chain, an optionally substituted $C_1$ to $C_{20}$ alkylaryl, an optionally substituted $C_1$ to $C_{20}$ alkylheteroaryl linker, a substituted or unsubstituted $C_4$ to $C_{12}$-membered aryl or heteroaryl linker, a polyacrylate radical comprising from 2 to 100 acrylate monomers, a polymethacrylate radical comprising from 2 to 100 methacrylate monomers, a polyether radical comprising from 2 to 200 ether monomers, and a copolymer having acrylate, methacrylate and/or ethylene moieties and comprising from 2 to 200 monomers.

8. A compound of formula I':

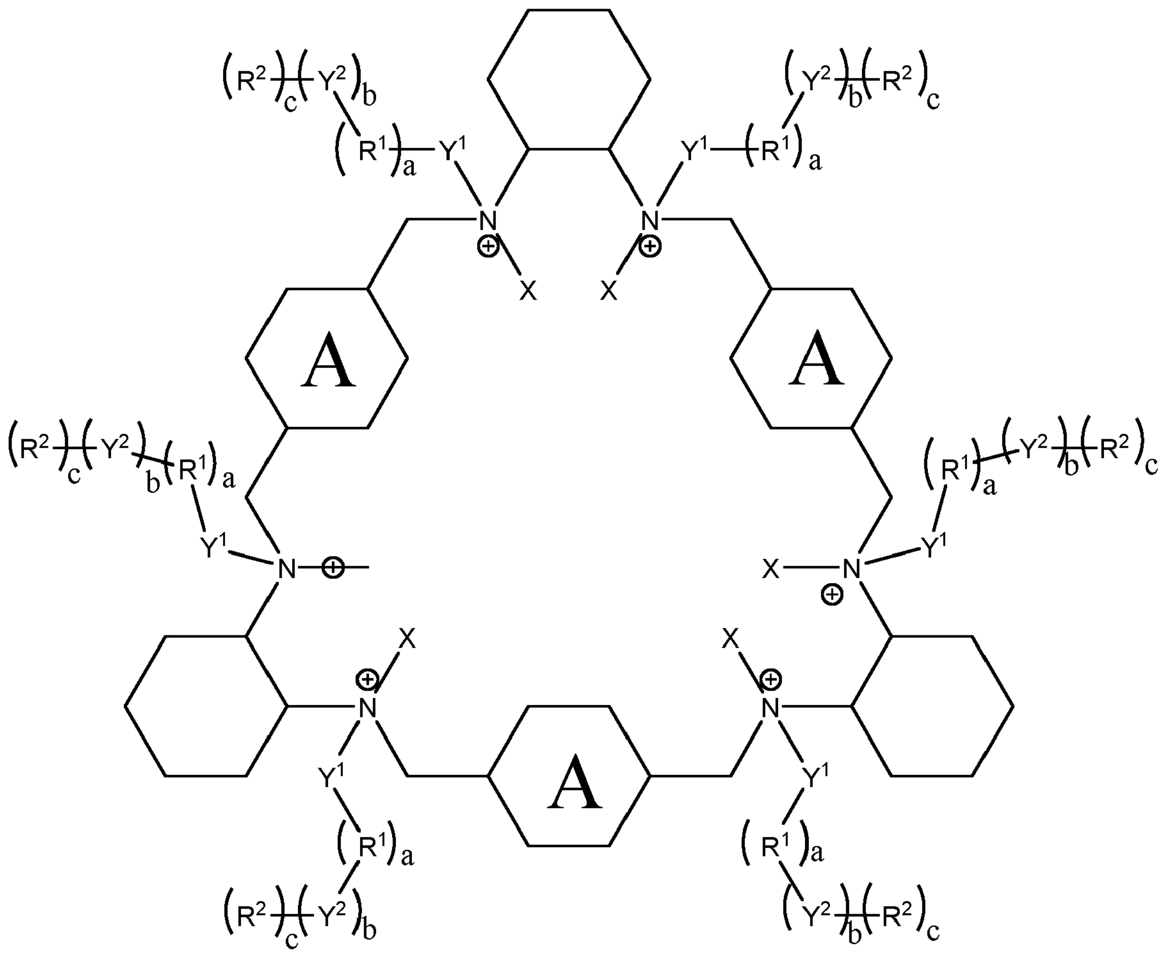

Formula I'

wherein,

- X is a halo group, preferably I, and A, $Y^1$, $R^1$, $Y^2$, $R^2$, a, b and c are defined as in previous claims.

**9.** A process of synthesis of a compound of formula I according to any of preceding claims 1 to 8, comprising a step of reacting a trianglamine of formula III:

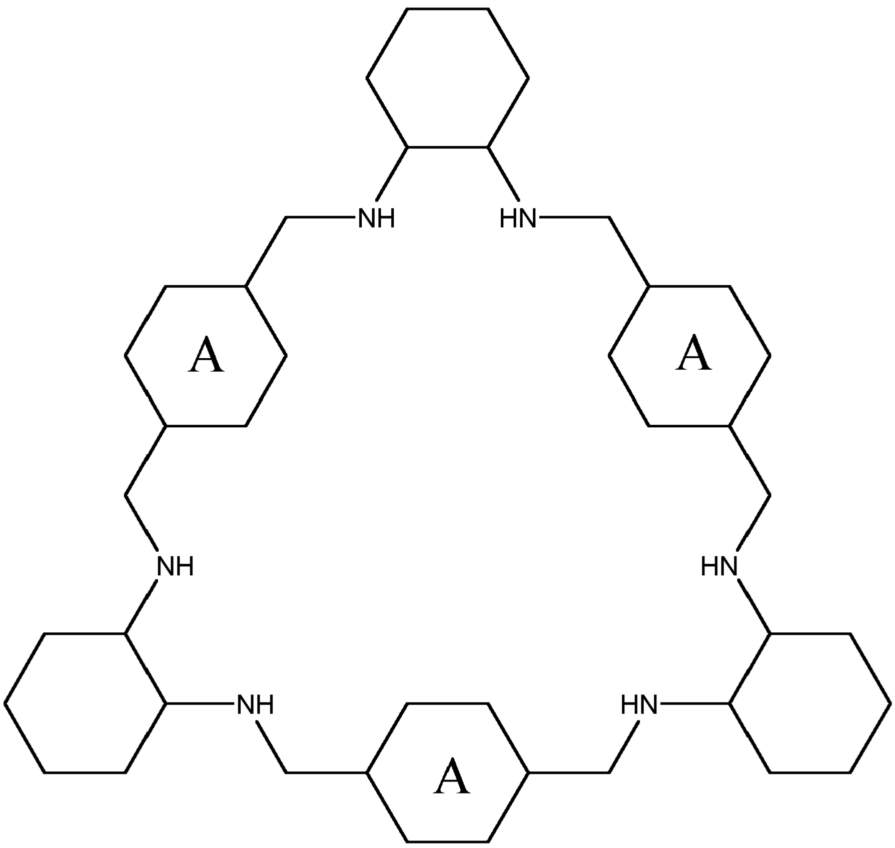

Formula III

with a compound of formula IV:

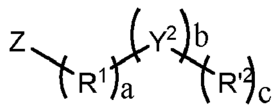

Formula IV

wherein,

- $R'^2$ represents a group of formula IV':

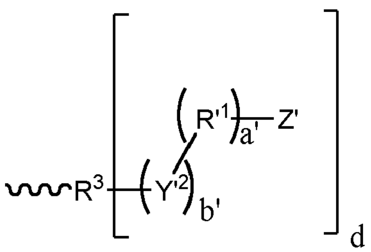

Formula IV'

- Z and Z', identical or different are chosen from isocyanate, acyle choride, acid, ester, halo and $\alpha,\beta$-unsaturated carbonyl, and
- A, $R^1$, $R'^1$, $Y^2$, $Y'^2$, $R^3$, a, b, c, d, a' and b' are defined in previous claims.

**10.** A process of synthesis of a trianglamine of formula III comprising the steps of:

a) reacting 1,2-diaminocyclohexane with a compound of formula V:

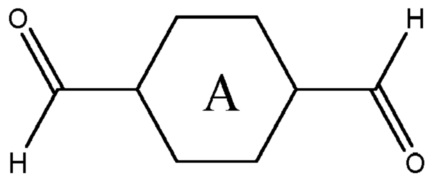

Formula V

wherein, A is defined as above,
b) reacting the compound obtained in step a) with $NaBH_4$, and obtaining the compound of formula III.

**11.** A process according to any of claims 8 to 9, comprising the steps:

a) reacting 1,2-diaminocyclohexane with a compound of formula V,
b) reacting the compound obtained in step a) with $NaBH_4$,
c) reacting the compound obtained in step b) with a compound of formula IV,

and obtaining the compound of formula I.

**12.** Use of a compound of formula I according to any of claim 1 to 8, for the decontamination of air, water or sea water, preferably from toxic material such as radioactive elements such as radionuclides, per- and polyfluoroalkyl substances, heavy metals, dyes and ions, iodine, the production of potable water via filtration, desalination of sea water and gas separation.

**13.** A gel comprising a compound of formula I according to any of claim 1 to 8 and a solvent.

**14.** A 3D ink comprising a compound of formula I according to any of claim 1 to 8, wherein $Y^2$ is acrylate or methacrylate, b = 1 and c = 0.

**15.** A membrane comprising a crosslinked compound of formula I according to any of claim 1 to 8.

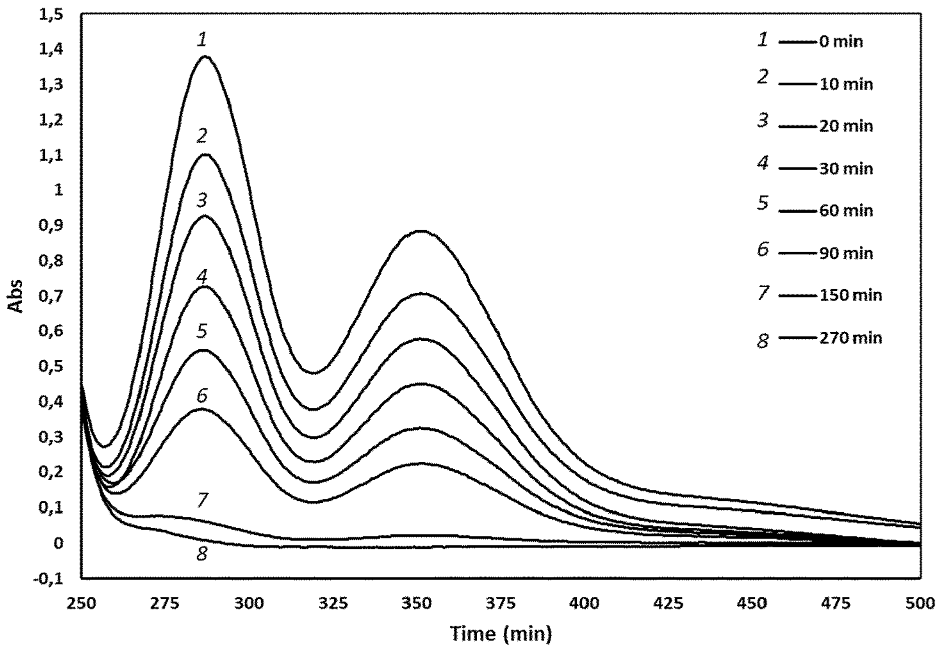


Figure 1

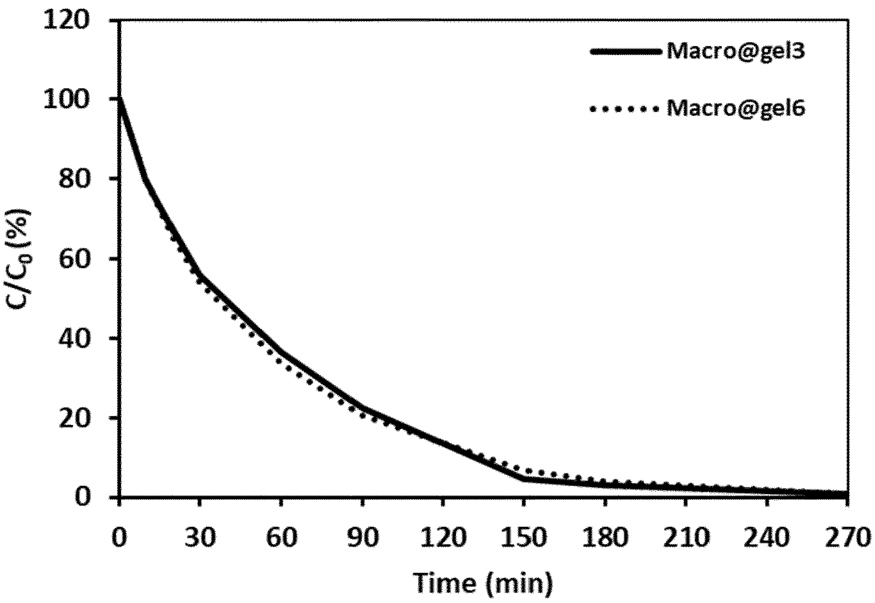


Figure 2

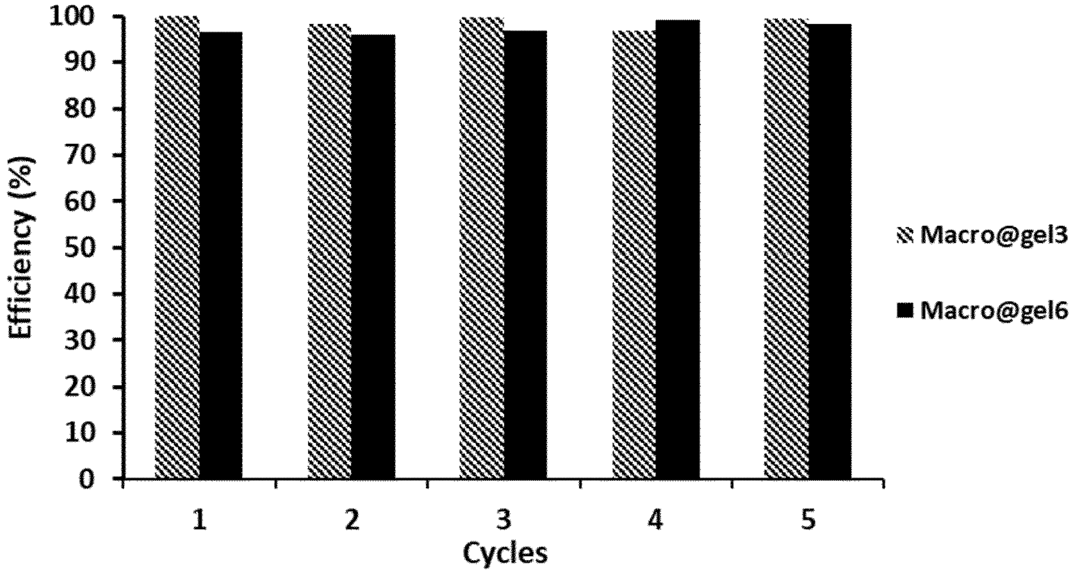


Figure 3

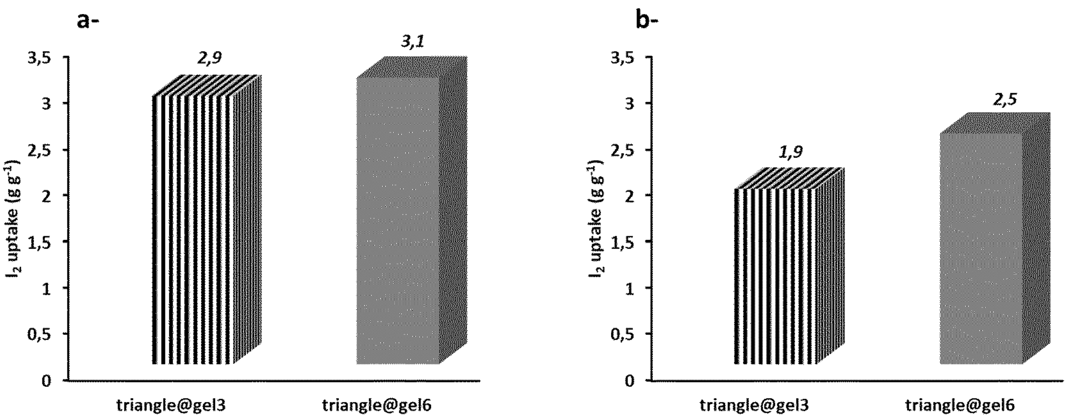


Figure 4

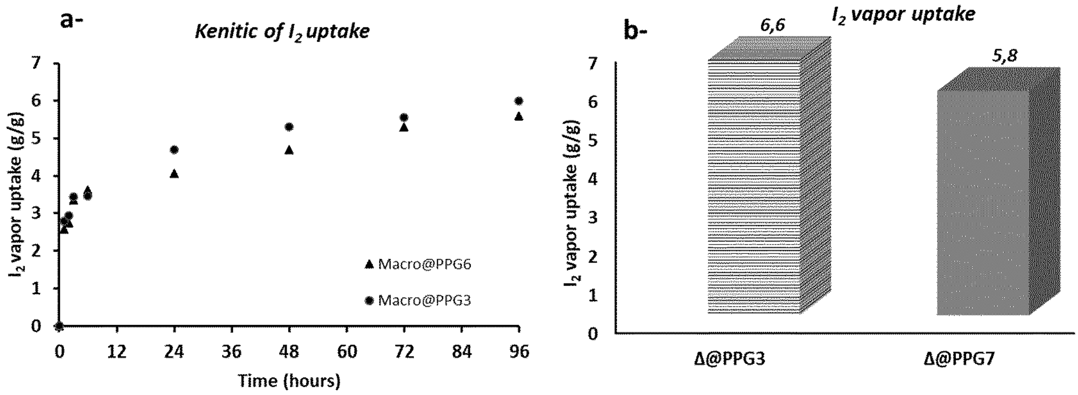


Figure 5

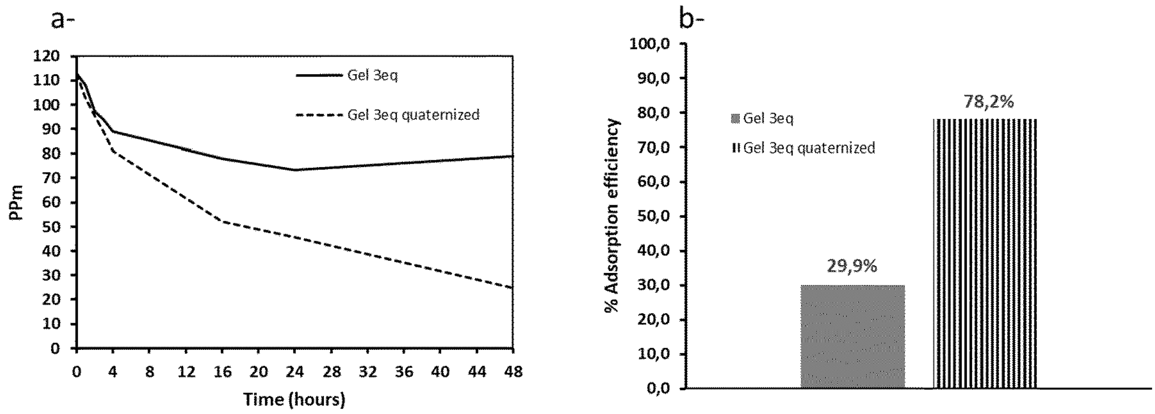


Figure 6

Europäisches Patentamt
European Patent Office
Office européen des brevets

# EUROPEAN SEARCH REPORT

Application Number
EP 22 30 7013

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X<br><br><br>A | WO 2005/030711 A2 (COVALENT PARTNERS LLC [US]; WHITEFORD JEFFREY A [US] ET AL.) 7 April 2005 (2005-04-07)<br>* page 35; compounds Hexamer 1jh-AC, 1dh-acryl *<br>* paragraph [0023] *<br>* claim 1 * | 1-4, 12-15<br><br>5-11 | INV.<br>B01D61/00<br>C07D259/00 |
| X | KUHNERT N ET AL: "Synthesis of enantiomerically pure functionalised trianglamine macrocycles by N-acylation and N-alkylation reactions", TETRAHEDRON LETTERS, ELSEVIER, AMSTERDAM , NL, vol. 47, no. 38, 18 September 2006 (2006-09-18), pages 6915-6918, XP025004897, ISSN: 0040-4039, DOI: 10.1016/J.TETLET.2006.06.160 [retrieved on 2006-09-18]<br>* compounds 2a-b, 4a-c * | 1-4 | TECHNICAL FIELDS SEARCHED (IPC) |
| X | JACEK GAWRONSKI ET AL: "Trianglamines-Readily Prepared, Conformationally Flexible Inclusion-Forming Chiral Hexamines", CHEMISTRY - A EUROPEAN JOURNAL, JOHN WILEY & SONS, INC, DE, vol. 12, no. 6, 30 November 2005 (2005-11-30), pages 1807-1817, XP071826491, ISSN: 0947-6539, DOI: 10.1002/CHEM.200500887<br>* compounds 1-11 * | 1-5 | C07D<br>B01D |

-/--

The present search report has been drawn up for all claims

3

| Place of search | Date of completion of the search | Examiner |
|---|---|---|
| Munich | 16 May 2023 | Miniejew, Catherine |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 30 7013

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KIRILA TATYANA ET AL: "Influence of Salt on the Self-Organization in Solutions of Star-Shaped Poly-2-alkyl-2-oxazoline and Poly-2-alkyl-2-oxazine on Heating", POLYMERS, vol. 13, no. 7, 2021, page 1152, XP093046491, CH ISSN: 2073-4360, DOI: 10.3390/polym13071152 * figure 1; compounds 1-4 * ----- | 1-4 | |
| X | PADMAJA M ET AL: "The synthesis of novel chiral macrocyclic and polymeric amines containing a trans-1,2-diaminocyclohexane system", TETRAHEDRON ASYMMETRY, PERGAMON PRESS LTD, OXFORD, GB, vol. 15, no. 15, 9 August 2004 (2004-08-09), pages 2437-2441, XP004525435, ISSN: 0957-4166, DOI: 10.1016/J.TETASY.2004.07.005 * compound 8 * ----- | 1-5 | TECHNICAL FIELDS SEARCHED (IPC) |

The present search report has been drawn up for all claims

3

| Place of search | Date of completion of the search | Examiner |
|---|---|---|
| Munich | 16 May 2023 | Miniejew, Catherine |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........................................................................................
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 22 30 7013

This annex lists the patent family members relating to the patent documents cited in the above-mentioned European search report. The members are as contained in the European Patent Office EDP file on The European Patent Office is in no way liable for these particulars which are merely given for the purpose of information.

16-05-2023

| Patent document cited in search report | Publication date | Patent family member(s) | Publication date |
|---|---|---|---|
| WO 2005030711 A2 | 07-04-2005 | EP 1667965 A2 | 14-06-2006 |
| | | US 2005154199 A1 | 14-07-2005 |
| | | US 2008290034 A1 | 27-11-2008 |
| | | US 2013081999 A1 | 04-04-2013 |
| | | WO 2005030711 A2 | 07-04-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 202258961 A
- WO 2022058961 A


### Non-patent literature cited in the description


- **B. VALIZADEH ; T. N. NGUYEN ; K. C. STYLIAN-OU.** *Polyhedron,* 2018, vol. 145, 1-15